(19) Europäisches Patentamt / European Patent Office / Office européen des brevets

(11) **EP 2 744 495 B1**

(12) **EUROPEAN PATENT SPECIFICATION**

(45) Date of publication and mention
of the grant of the patent:
**18.01.2017 Bulletin 2017/03**

(21) Application number: **12772805.3**

(22) Date of filing: **14.08.2012**

(51) Int Cl.:
*A61K 31/425* (2006.01)     *A61K 31/7048* (2006.01)
*A61P 33/10* (2006.01)     *A61K 47/22* (2006.01)
*A61K 9/00* (2006.01)     *A61K 45/06* (2006.01)
*A61K 31/365* (2006.01)     *A61K 31/429* (2006.01)

(86) International application number:
**PCT/IB2012/054120**

(87) International publication number:
**WO 2013/030702 (07.03.2013 Gazette 2013/10)**

(54) **ANTHELMINTIC FORMULATIONS AND TREATMENTS**

ANTHELMINTHIKUM ZUBEREITUNGEN UND BEHANDLUNGEN

COMPOSITION ANTIHELMINTIQUE ET TRAITEMENTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.08.2011 NZ 59461011**

(43) Date of publication of application:
**25.06.2014 Bulletin 2014/26**

(73) Proprietor: **VIRBAC
06516 Carros (FR)**

(72) Inventors:
- **CUFF, Nicola Jane
 Auckland 2013 (NZ)**
- **PULFORD, Peter Norman
 Auckland 2013 (NZ)**
- **VICKERS, Mark Colin
 Auckland 2013 (NZ)**

(74) Representative: **Gevers & Orès
41 avenue de Friedland
75008 Paris (FR)**

(56) References cited:
**WO-A1-00/74489     WO-A1-2004/009080
WO-A2-2008/075984     US-A- 6 013 636
US-A1- 2007 042 013**

- **LE JAMBRE L F ET AL: "Characterization of
 moxidectin resistant Trichostrongylus
 colubriformis and Haemonchus contortus",
 VETERINARY PARASITOLOGY, ELSEVIER
 SCIENCE, AMSTERDAM, NL, vol. 128, no. 1-2, 10
 March 2005 (2005-03-10), pages 83-90,
 XP027655471, ISSN: 0304-4017 [retrieved on
 2005-03-10]**

EP 2 744 495 B1

## Description

### Technical Field

This invention relates to the use of anthelmintics, anthelmintic formulations, and novel methods of controlling nematode parasitism in ruminants (particularly cattle and sheep).

### Background Art

Internal parasites of grazing animals especially sheep, goats, and cattle are a direct threat to the economics of meat production as the internal parasites especially worms (helminths) rob the young animal of nutrients, reducing potential weight gain.

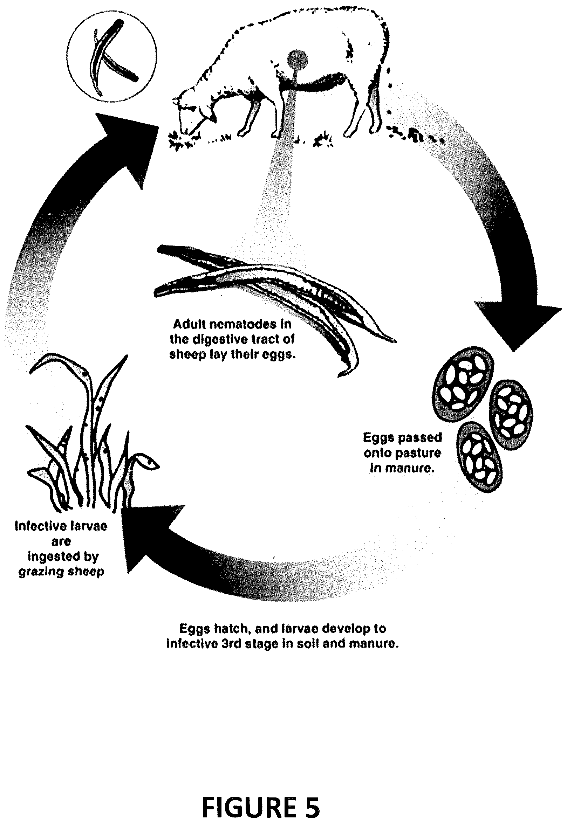

The life cycle of worms is shown generally in Figure 5 which explains how infective larvae are ingested by sheep; the larvae become adults within the sheep and lay their eggs in the sheep's digestive tract which are then passed to the pasture. The eggs hatch outside the sheep and the larvae develop to the infective 3rd stage in soil and manure. The larvae are then ingested by sheep and the cycle repeats itself.

### Disclosure of Invention

### Technical Problem

### The Basic Lifecycle of the Major Groups of Nematodes

The lifecycles of the parasitic species vary considerably, as would be expected from such a large and diverse group. There are however a number of common features. Firstly, the parasite undergoes a series of moults through larval stages (designated L1 to the adult L5 form). Secondly, in most (but not all) nematodes it is the L3 larvae that is the infective form, important exceptions to this being the Ascarids, such as Ascaris lumbricoides and the pinworms, where it is either the L1 larvae, or eggs containing L1 or L2 larvae that are infective. Thirdly the L3 form onwards in all species undergoes a migration within the body of the definitive host as it matures into the adult parasite, usually via the bloodstream or lymphatic system to the heart, lungs, trachea, and then to the intestine. Finally, in most cases the parasite leaves the definitive host as thin walled eggs in the faeces.

A first stage larva develops inside an egg, then hatches. Initiation of the hatching process is controlled by several factors including temperature and moisture levels in the external environment. Hatching occurs only when environmental conditions are favourable for survival of hatched larvae. These conditions stimulate the enclosed larvae to assume its own role in hatching by secreting enzymes to digest the surrounding egg membranes, then exerting pressure against the weakened membranes to emerge therefrom.

Nematodes moult four times during each life cycle with a moult occurring at the end of each larval stage. Therefore, moults separate the first and second larval stages (L1 and L2), the second and third larval stages (L2 and L3), the third and fourth larval stages (L3 and L4) and also the fourth larval stages and immature adults (L4 and L5). The L5 grows to the limit of its new cuticle, at the same time developing into a sexually mature male or female adult.

By way of example when sheep were introduced into New Zealand in the late 19th century they brought twenty-nine species of internal parasites with them. Ever since, internal parasites have been the cause of sheep-health concerns for farmers. Management of the sheep, the pasture they graze on and the climate form an integral part on how the lifecycle of the internal parasites can continue and the levels of pasture contamination, and thus the infection in ewes and lambs.

Generally there will be a build-up of parasites in spring and autumn, with lower levels in the summer because of dryness, and in the winter because of lower temperatures. Everywhere in New Zealand the pattern of the infection is about the same, with the different species of parasites having a peak period slightly staggered right through the seasons. Larval numbers on pasture will build up during spring and summer, reaching a peak in autumn. It's the remnants of this autumn peak that survive the winter and will start the new lifecycle and be the major source of infection of the new lambs the following spring. Lactating ewes in some years (but not every year) add to the larval population and contaminate the pasture.

The species that bother sheep the most are Haemonchus contortus (barbers pole worm), Ostertagia, Trichostrongylus and Cooperia. In the examples we have studied Cooperia and Ostertagia in cattle and we have further supporting evidence of lungworm (Dictylocaulus viviparous) in calves. Under favourable conditions these species can suppress normal production and even cause death in cattle and sheep. Mortality rates in non-drenched lambs can reach 10 to 45% in the first year of life. The sub-clinical effects are a significant reduction in wool production, live weight gain, and fertility in later life, quite often without further outward signs.

Lambs will remain susceptible to worm infections in the first year of their life, but they develop immunity at 10 to 12 months old that generally limits worm burdens. This immunity will remain with them for the rest of their lives. Sheep need to be regularly exposed to worm challenges so that this immunity can be maintained. It can fail at times of stress because of severe feed shortages and the well-known decline in immunity of ewes at lambing time. By week four after lambing, this immunity is largely restored.

Lambs that have not been drenched provide the main source of contamination of the pasture. Even when drenched, lambs remain a significant source for nematode eggs and become readily re-infected between drenching. This can be avoided by increasing the frequency of drenching at danger periods or by the use of slow release capsules. Since it is not practical or possible to eradicate all populations of parasites at an acceptable price, the aim of control measures is to keep parasites at a level that is economical and viable for sheep production. Nutrition has an important influence on the development and maintenance of immunity in sheep and on its performance, and thus, on the effects of parasites. The reduction of stress by feeding high protein supplements to lambing and lactating ewes has been shown to give a large reduction in worm burden faecal egg counts (FECs), thus eliminating the need for the use of anthelmintics.

Drench resistance is a real threat to worm control in goats, sheep and cattle. About 80% of milking goat herds and 65% of sheep flocks may already be affected, and on some goat and sheep farms, resistance to all three common drench families (Benzimidazoles, Levamisole/Morantel, Macrocyclic lactones) has been recorded.

Frequent drenching although providing a short term relief can acerbate the long term problem of the build-up of resistant worms in the pasture and hence infection of lambs and other young animals. Sheep and cattle less than 9 months old are the major contributors to internal parasite populations.

With resistant worms in the pasture even a long wet summer with extra grass growth may make it difficult to fatten lambs despite the abundance of feed. It appears that the resistant worm population may expand to negate any possible weight gain in the young animals.

Apart from pasture management and grazing of "clean" pasture, one authority recommends the following steps for farmers to reduce the build-up of resistant worms:

a) Avoid excessive or unnecessary drenching;
b) Do not under-dose;
c) Get drench effectiveness checked (see below); and
d) Prevent the introduction of resistant worms from elsewhere.

Farmers are recommended to arrange for a faecal egg count (FEC) test about ten days after drenching to check that the drench is working.

Various steps have been taken to control the worm burden in cattle and sheep, and much work has been carried out in the development of combination anthelmintic formulations involving two or more actives, due to worm populations becoming resistant to treatment with single actives.

**Prior Art**

Generally, as drench resistance increases, the period between treatments reduces, or new anthelmintics or new combinations of anthelmintics are tried. Different classes of anthelmintics can be difficult to formulate together, and some combinations are incompatible, and degrade if held in the same solvent formulation.

Examples of combination treatments include:

US 4159337 26 Jun 1979

US 4166858 4 Sep 1979

WO 19941028887 22 Dec 1994

WO 2004/069242 19 Au 2004

NZ 336139/ 336213/ 336814 , WO2004/009080 and WO00/74489

Pour-ons while convenient to use, require at least twice the dose of an oral or injectable formulation to pass through the skin in effective amounts, and there is large between animal variability in how the anthelmintic gets through the skin, and this varies with the animals individual skin type and also seasonally and also can be influenced by rain. Additionally animals can lick off the pour-on and vary the dose that an animal receives.

**Solution to Problem**

**Technical Solution**

It is an object of this invention to provide improved controlled strategies for the control of internal parasites in ruminants, and/or to provide a novel formulation particularly suited to the control of nematode parasitism in cattle and sheep, or ones which will at least provide the public with a useful choice.

**STATEMENT OF THE INVENTION**

In one aspect the invention resides in the use of levamisole and one or more macrocyclic lactones (MLs) in the manufacture of a veterinary composition or compositions for the treatment of a young ruminant animal at intervals of 40 to 60 days by allowing the animal's immune system to recognise incoming larvae as foreign.

In a related aspect the invention resides in the use of levamisole and one or more macrocyclic lactones (MLs) in controlling internal parasites in ruminants and extending the time between treatments to minimise drench use, by treating the animal with levamisole to substantially eliminate the initial adult and immature parasite population present in the animal, and at substantially the same time treating the animal with one or more doses of a long acting ML that acts for a duration of a minimum of about 3 to 5 days uor more to re-program the animal's immune system so that the animal can recognise incoming larvae as foreign and mount an immune response to the incoming larvae.

We have found that oral formulations of short acting ML's such as oral ivermectin with less than 3 days activity do not appear suitable and only formulations with 3-5 days or more persistent activity appear suitable to consistently produce this response. Hence we recommend the use of ML's with persistent activity of at least 3-14 days, preferably as long as 7-14 days and optimally around 10-14 days, and not more than 50 days.

Preferably the long acting ML has a persistent activity of about 10-14 days for Cooperia (but for other worm species it is often longer). We have found that a persistent activity of 7 days works but 10-14 days is better for Cooperia, and 21 days (as with the Eclipse pour on) did not give any better response. We believe this was the result of trying a pour-on rather than an injectable formulation.

In some cases we use a longer persistent activity such as 21-35 days for some worms species as the larvae from different worm species vary in their innate sensitivity to an ML so may come in later, lungworm for example are highly susceptible so come in later - this is shown on the Ivomec Injection shown below, 7 days for Cooperia, 21 days for Oesophagostomum and lungworm.

Preferably this is a long acting oral or injectable or implantable ML formulation.

The ML is typically ivermectin or abamectin though any of the macrocyclic lactones (ML's) can be used.

Preferably the release profile of the ML formautions is such that it will have persistent activity for up to 3 to 5 or more days with its role to remove/kill incoming larvae for a sufficiently long period to allow the host to recognise incoming larvae as foreign and mount an immune response/self-treatment effect.

The minimum persistent activity (also called the "treatment period") for the long acting ML can vary depending upon the route of administration and the nature of the ML used. For example if the long acting ML is an oral formulation then the shortest typical treatment period could be 2 to 3 days. In the case of an injectable formulation of the long acting ML the shortest typical period is more likely to be 3 to 5 days for an abamectin injection and from 5 to 7 days for an Ivomec injection. An implantable ML treatment is likely to have a persistent activity of up to 5 to 14 days (more preferably 10 to 14 days or longer).

Hence (a) an oral treatment could give the desired persistent activity of 3 to 14 days (more preferably 10-14 days), (b) the injectable treatment could give a persistent activity of up to 3 to 14 days (more preferably from 10-14 days) and (c) the implantable ML treatment is likely to have a persistent activity of up to 3 to 14 days (more preferably 10-14 days).

Preferably the treatment regime involves an initial dose of levamisole base or levamisole phosphate at about 3-9mg/kg levamisole base equivalent and more preferably about 7.5 mg/kg of the animal's live weight and an initial dose of the long acting ML in the dose range of 0.1-0.5mg/kg, and more preferably at about 0.20 mg/kg of the animal's live weight and repeating the treatment at intervals of 40 to 60 days.

In the case of levamisole a higher dose than 9mg/kg is less desirable in an injection because of toxicity. On the other hand at a dose of 3mg/kg or below you run the risk of under-dosing with levamisole for Cooperia. A dose rate of 7.5mg/kg appears to be optimum to achieve the immune effect

However in the case of the ML you can double the dose of an ML so a dose range of 0.1-0.5mg/kg could be given, a dose lower than 0.1mg/kg is undesirable for efficacy but a higher dose is not.

In another aspect the invention resides in a method of controlling parasites in ruminants by treating a ruminant animal with at least (a) a long acting oral or injectable or implantable anthelmintic formulation (formulation A) capable of removing or killing incoming larvae for a sufficiently long period to allow the host to recognise incoming larvae as foreign and mount an immune response to the incoming larvae, and (b) treating the ruminant with a short acting oral or injectable anthelmintic formulation (formulation B) to substantially eliminate the initial parasite population.

Preferably the two formulations A and B are separate injectable formulations administered to the animal, and wherein formulation B is injected into the animal at the same time as or before formulation A.

Preferably formulations A and B are delivered simultaneously to the animal in a combined injectable formulation.

Preferably the short acting anthelmintic formulation B contains levamisole and is capable of peaking within 1-8 hours, and being substantially eliminated from the ruminant within 48 hours of treatment.

Preferably the long acting anthelmintic formulation A contains an active chosen from the class of macrocyclic lactones and is capable of providing a sustained release of anthelmintic for 3-14 days.

Preferably the animal is treated with about 7.5 to 8 mg/kg of levamisole base and about 0.20 mg/kg of a macrocyclic lactone. This can be contrasted with the rate used for ML pour-ons which is typically 0.5mg/kg of the ML. Typically the macrocyclic lactone is abamectin.

In the case of the Ivomec injection (which we have tested) the concentration of the ML is at 10g/1000ml or 1.0%.

The preferred range for the ML as used in this invention whether in a single injectable or as a separate injection is from 0.1-2%w/v of the formulation, and more preferably from 0.5% to 2.0% w/v of the formulation. However the amount in the formulation is not as important as the dose rate of the ML delivered to the animal - which is preferably from 0.1 to 0.5mg/kg of the animal's live weight.

The preferred range for the levamisole base is from 5% to 60% w/v of the formulation and more preferably about 37.5% w/v. However the amount of levamisole base in the formulation is not as important as the dose rate of the levamisole base delivered to the animal - which is preferably from 3 to 9 mg/kg and more preferably 7.5mg/kg of the animal's live weight.

These concentrations can vary as it is the mg/kg dose rate that is critical and the precise concentration of the formulation may vary, as the dose volume given can be adjusted to allow these dose rates being achieved in the animal.

Preferably the ruminant animal is not treated again with the treatment (as above) until at least about 40 days has elapsed since the first treatment.

The treatment regime of this invention allows for longer treatment intervals of from 40 to 60 days compared to the shorter recommended treatment intervals of 28 days for other known products such as Arrest C.

In a further aspect the invention may be said to reside in a method of controlling parasites in ruminants and extending the time between treatments to minimise drench use, by treating the animal with at least (a) a long acting anthelmintic formulation (formulation A) capable of removing or killing incoming larvae for a sufficiently long period to allow the host to recognise incoming larvae as foreign and mount an immune response to the incoming larvae, and (b) a short acting anthelmintic formulation (formulation B) to substantially eliminate the initial adult and immature parasite population present in the animal and (c) an immuno-stimulating or immuno-modulating agent that may or may not be one of the anthelmintics.

Levamisole is a known immuno-modulating agent that has positive effects on the immune system of a wide range of animals from fish to mammals including man. The applicant has also observed that after anthelmintic treatment in accordance with the invention other conditions such as ringworm on the skin of calves (a disease that is known to have in part an immunological basis for self-cure) also appears to reduce (both in size, number and severity) after treatment, while in untreated calves it did not.

This invention is based on the breakthrough that by keeping the young animals substantially free of internal parasites by the suitable anthelmintic killing any resident adult internal parasites of a particular species and preventing the development of the larvae of that species for a sufficiently long period of time, and in the presence of an immuno-modulating and stimulating compound, the animal's immune system is effectively re-programmed so that when larvae are ingested from the pasture and begin to reinfect, the animal's immune system recognises them as "foreign" and deals with them accordingly. This also has the advantage that the build-up on the pasture of drench resistant larvae is minimised.

The minimising of infective larvae on pasture is particularly useful. High pasture contamination can not only lead to very heavy worm burdens in animals that graze the pasture, which then leads to disease, but it has also been shown that even in an "immune" animal, for the animal to control these stages there is an energy cost and therefore there is reduced energy for growth, or production such as wool or milk or reproduction.

The rapid development of high worm burdens also requires treatment to be given more frequently, while slow development of burdens lengthens the retreatment interval and reduces anthelmintic use which also slows the development of anthelmintic resistance.

The applicant has demonstrated the difference between treated and untreated calves in farms with macrocyclic lactone resistant Cooperia. The difference in egg output and contamination between untreated animals, those treated with ineffective single active long acting macrocyclic lactone drenches, and those treated with the formulations of the invention is vast (see table and graph - insert page number reference or graph and table numbers?). The beneficial effect of treatment with the formulation of the invention is evident not only 28 days after treatment, but low egg counts continued in calves treated with the anthelmintic of the invention to 31 days and has even been observed up to 55 days after treatment, which is long after re-infection and high egg output could have occurred.

The period of re-infection to egg output (called the "prepatent period") for Cooperia and Ostertagia is approximately 17-21 days, and typically long acting ML drenches do not have persistent activity against incoming larvae of 7-14 days for Cooperia.

It was also noted in a lungworm outbreak in calves that those treated with the formulation of the invention at the start of the outbreak showed no evidence of clinical disease for at least 95-100 days (despite evidence of lungworm infection establishing at low levels in these calves at about 55 days after treatment) while in contact calves on the same pasture and treated with short acting anthelmintics (oral benzimidazole and levamisole) all showed clinical respiratory signs and death 14 to 17 days after oral treatment.

Susceptibility to lungworm has also been shown to have an immunological basis, with older animals becoming immune, and young calves such as those observed above often dying when exposed to very high pasture larval challenge, and often before the lungworm have time to mature and produce eggs/larvae by approximately 28 days after infection. Those calves that survive often have very severe lung damage that resolves slowly.

Studies by the applicant have also shown that the adult stages of Cooperia spp that reside in the gut of calves may have a high level of resistance to anthelmintics such as to the macrocylic lactones family, preventing removal of these "resistant" stages and therefore these continue ongoing egg production and contamination of pasture. These worm eggs that contaminate the pasture develop and moult to free living pasture stages (L1, L2), becoming an infective larval stage (L3) that go on to orally re-infect the animals grazing this contaminated pasture, and complete their life cycle by further moulting to become an L4 larvae and then finally an adult stage (L5) within the animal.

These larval stages have a different physiology and studies by the applicant have shown that these immature stages do not show the same resistance to drench families such as the macrocyclic lactones, which allows a long acting active formulation of a macrocyclic lactone to prevent the reestablishment of the infective larval stage, and therefore stop the lifecycle of the worm and prevent worm egg production, if the resistant adult stages are completely removed.

As the larval stages are still sensitive, long acting macrocyclic lactone formulations have been found by the applicant to continue killing incoming larvae stages by the anthelmintic alone for 7-14 days and sometimes longer. If however the animal becomes more immuno-competent, a similar process of preventing establishment of infective larvae (as well as other methods of limiting worm burdens) can occur by the animal itself sometime after this. Studies have shown that a small numbers of calves in a population have the ability to better control worms such as Cooperia spp by killing incoming larvae, shortening and stunting the adult stages, reducing egg output and egg counts, and progressively moving established worms further down the intestinal tract to assist in their natural removal.

The inventive step is therefore this new way of re-programming the animal's immune system which enhances this natural process in a greater proportion of the population and reduces the reliance on the anthelmintic alone and the need for intensive drenching.

In another aspect the invention can be said to be the use of levamisole and one or more macrocyclic lactones (MLs) in the manufacture of a veterinary composition or compositions for the treatment of a young ruminant animal to extend the time between treatments by allowing the animal's immune system to recognise incoming larvae as foreign and assist in their removal.

Preferably the short acting anthelmintic formulation (formulation B) is capable of peaking within 1-8 hours, and being substantially eliminated from the ruminant within 48 hours of treatment.

More preferably the short acting anthelmintic formulation (formulation B) comprises levamisole and is preferably provided as an oral or injectable formulation.

The reference to "long acting anthelmintic formulation (formulation A)" and "short acting anthelmintic formulation (formulation B)" is intended to cover separate formulations containing actives A or B, as well as single formulations containing both actives A and B (and the reference to "formulations" in the latter case should be read as applying to the actives present within the single formulation).

Most preferably the formulation is an injectable formulation containing both actives.

In this aspect the invention provides an injectable veterinary formulation comprising a macrocyclic lactone in the range of from 0.5% to 2% w/v, and levamisole in the range of from 5% to 60% w/v, dissolved in a non-aqueous solvent system.

The levamisole may be present as levamisole hydrochloride or levamisole phosphate, but more preferably the levamisole is present as levamisole base.

(Levamisole base is used in the production of levamisole hydrochloride. It is not usually present in levamisole formulations.)

Preferably the non-aqueous solvent system includes or comprises one or more solvents chosen from the group comprising: N-methyl-pyrrolidone, N-pyrrolidone, glycerol formal, triacetin, ethanol, 1-propanol, 2-propanol, 1-butanol, 2-butanol, 1-pentanol, 2-pentanol, 3-pentanol, propylene glycol, PEG 200, PEG 300, PEG 400, diethylene glycol ethyl ether, isopropylidene glycerol, dimethyl isosorbide, propylene carbonate, glycerol, acetone, methylethylketone (MEK), dimethylsulfoxide (DMSO), 1-dodecylazacycloheptane, dipropyleneglycol methyl ether, methyl acetate, ethyl acetate, ethyl lactate, dimethylformamide, N,N-diethyl-m-toluamide, dimethylacetamide, ethylacetamide, tetrahydrofuran, caprolactam, decylmethylsulfoxide, solketal, propylene carbonate, ethyl lactate and mixtures thereof.

More preferably the non-aqueous solvent system includes or comprises one or more solvents chosen from the group comprising: N-methyl-pyrrolidone (abbreviated to "NMP"), N-pyrrolidone, glycerol formal, and triacetin.

In its most preferred form according to some of the examples the non-aqueous solvent system comprises glycerol formal.

(Glycerol formal is a mixture of two isomers, 4-hydroxymethyl-1, 3-dioxolane and 5-hydroxy-1, 3-dioxane. These isomers are present in a constant ratio of 40% to 60% w/v respectively.)

Preferably the levamisole is in the range of from 30% to 40% w/v.

Preferably the levamisole is present in an amount of about 37.5% w/v.

Preferably the macrocyclic lactone is present in an amount of about 1% w/v.

Preferably the macrocyclic lactone is chosen from the group of avermectins.

Preferably the macrocyclic lactone is chosen from the group comprising abamectin, moxidectin, doramectin, ivermectin, and eprinomectin.

More preferably the macrocyclic lactone is abamectin.

Preferably the injectable formulation also includes one or more stabilisers and/or preservatives.

Optionally the formulation may also include one or more other anthelmintics such as a benzimidazole.

More preferably formulation B is designed to peak within about 2-4 hours of administration, and preferably is formulated so as to be substantially eliminated from the ruminant within 24-48 hours of treatment.

The primary role of this short acting anthelmintic formulation (formulation B) is to virtually eliminate or assist in the removal of the initial resident worm population in the animal host. A secondary role of anthelmintic formulation B is to act as a host immune stimulant, and levamisole appears to provide both functions.

In another aspect of the invention the dose rate of formulation B can be lowered without compromising either the worm kill (efficacy) or immune stimulation, allowing for more easy formulation of a combined anthelmintic injection or implant.

Preferably the active in the long acting anthelmintic formulation (formulation A) is chosen from the group of macrocyclic lactones.

More preferably formulation A is provided in the form of a long acting injectable liquid formulation, but it may also be provided as an implant formulation and there are numerous known delivery mechanisms that can be used in order to provide the ruminant with a long acting (persistent) or sustained release formulation. It is preferable that this long acting formulation is capable of providing a sustained release of the anthelmintic for at least 3 days. We have found that from 3 to 5 days to about 14 days is enough to allow the animal's immune system to be re-programmed to then recognise future incoming larvae as "foreign".

More preferably the long acting anthelmintic (formulation A) is present in the host for about 10-14 days, to enable the anthelmintic to remove or kill incoming larvae for a sufficiently long period of time to allow the host to recognise the incoming larvae as foreign, and thus allow the host to mount an immune response against these incoming larvae.

These two formulations can be supplied as separate formulations, and whilst this is easier for the formulator because of the difficulty in formulating a combination of incompatible actives such as macrocyclic lactones and levamisole, it is less desirable from the point of view of the farmer who has to administer two separate formulations typically by two separate injections. The two different actives in the formulations need to have completely different release profiles, and thus it is possible that the method of this invention may involve two separate injections of these two different formulations, preferably at about the same time, although the timing could be staggered, as in one version of the invention it may be preferable to provide the short acting anthelmintic (formulation B) (such as levamisole) by way of an injection, about 4-8 hours before the introduction of the long acting anthelmintic (formulation A) (such as a macrocyclic lactone).

Conversely the long acting anthelmintic formulation (formulation A) may be supplied to the animal before the short acting anthelmintic (formulation B), although this is less preferable, and for the farmers convenience it is preferable that the two different formulations are supplied at about the same time.

Best of all the two actives can be formulated as a single injectable composition making it easier for the farmer to inject both actives at the same time at the required dose rates.

In another aspect the invention provides the use of an anthelmintic formulation combining a long acting anthelmintic formulation (formulation A), preferably containing a macrocyclic lactone, capable of a sustained release over a period of from about 10 to about 14 days, together with a short acting anthelmintic formulation (formulation B) preferably containing levamisole, capable of peaking within 1-8 hours of administration and designed to be substantially eliminated from the animal within 24-48 hours of treatment.

The preferred form of the invention provides the use of a single injectable formulation that contains 37.5% w/v of levamisole base and 1% w/v of a macrocyclic lactone such as abamectin so that it is possible to provide a dose of 1mL/50kg of live weight of animal, which delivers 7.5 mg/kg of the levamisole base and 0.20 mg/kg of the macrocyclic lactone. Those skilled in the art also recognised that the dose rate in mg/kg to the animal is the critical step and that more concentrated (lower dose volume) or more dilute formulations (higher dose volume) could be used to give the same dose rate in mg/kg.

We have found that pour-on formulations are not suitable for this invention as they cannot be relied upon to deliver the required amount of levamisole effectively. We have shown that we need to provide rapid high levels of levamisole to be effective, and pour-ons do not always deliver this and they are influenced by weather, blood supply to the skin and the like.

These and other aspects of this invention, which should be considered in all its novel aspects, will be apparent from the following description which is given by way of example with reference to the following examples and to the following drawings.

## BRIEF DESCRIPTION OF THE DRAWINGS

Figure 1 is a graph of the results from the comparative study described in the Example 2.

Figure 2 shows total larvae recovered in culture per 25g by treatment group.

Figure 3 is a graph of the blood plasma profile for both levamisole and abamectin taken from the formulation of Example 1.

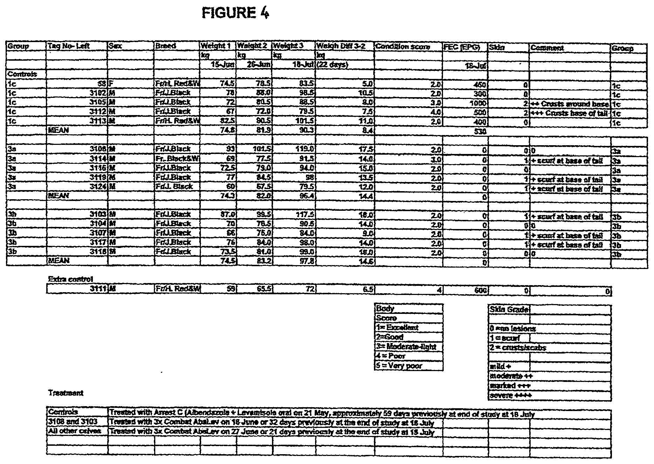

Figure 4 is a comparative table of trial results between the applications of 3 x recommended label amount of the formulation of Example 1 (called in the table "Combat AbaLev").

Figure 5 shows the life cycle of nematodes.

**Best Mode for Carrying out the Invention**

**Best Mode**

## DETAILED DESCRIPTION

The applicant has discovered that the presence of levamisole in such a method of treatment will also enhance the animal's immune system, making the animal better able to resist and respond to incoming larvae.

It is difficult to formulate levamisole with other actives in an injectable formulation. If it is prepared in an aqueous formulation, then the levamisole has to be kept below a certain pH in order to prevent the formulation from becoming unstable. On the other if it is formulated in oils, it is likely that the formulation will create an inflammation at the injection site, which is painful to the animal and suggests that the formulation is not well tolerated by the animal.

There is thus a need for a new formulation that will enable levamisole to be prepared in an injectable form with at least one other anthelmintic, and preferably an anthelmintic chosen from the class of macrocyclic lactones - refer to Examples 3 onwards.

**Definitions**

| Helminths | wormlike organism that live and feed off living hosts. Those that live inside the digestive tract, thoracic and abdominal organs are called internal parasites |
| --- | --- |
| Worm burden | the number of worms an individual host carries |
| Nematodes | various species of roundworm (phylum Nematoda) many of which infect ruminants especially cattle and sheep |
| FEC | Faecal Egg Count. This is a technique that takes a sample of fresh dung from the animal, and their eggs are extracted, and counted via a microscope to then calculate the number of eggs per gram (EPG) in the faeces, to determine the worm burden of the animal. |
| ML | stands for macrocylic lactone. Examples include abamectin and ivermectin, although his is a large class of anthelmintics. |

**A short acting ML** is an ML such as ivermectin oral which has no significant persistent activity against gastrointestinal roundworms.

**A long acting ML** is anything that has persistent activity of 7 days or longer, preventing significant re-establishment of larvae for this time. (Note that in NZ and Australia registration for a product with less than 7 days activity cannot be registered for persistent activity, activity needs to be 7 days or longer.

Levamisole is available in a number of different salts. Typically formulations contain levamisole phosphate, or levamisole hydrochloride. Whatever salt is used the dose rate of levamisole is calculated as the equivalent amount of the levamisole base.

**Example 1**

The inventive concept is an entirely new strategy to control nematode parasitism in ruminants (e.g. cattle, goats and sheep) which in part uses the host's own immune system. This method of treatment of ruminants and the formulations designed for this purpose would be a means to minimise drench use by increasing the time between treatment intervals, and it is believed will also stimulate and make use of the hosts own immune system.

The invention can best be understood by considering the two parts and their functions.

One part is a long acting injectable liquid (or implant) anthelmintic such as a (ML) typically ivermectin, or abamectin though any of the macrocyclic lactones (ML's) can be used (and possibly another anthelmintic type) that act to release for 3 to 5 or more days with its role to remove/kill incoming larvae for a sufficiently long period to allow the host to recognise incoming larvae as foreign and mount an immune response/ self-treatment effect. The other part is a short acting injectable liquid (or implant) containing levamisole with a kinetic profile similar to an oral or injectable formulation (e.g. peaking about 2-4 hours and virtually eliminated by 24-48 hours) to act to assist removal of the initial resident worm population and also act as a host immune stimulant.

The MLs are fat soluble and go to body fat and then are released over this time. Oral moxidectin in sheep is actually quite persistent and while "released "immediately (it is absorbed within 24 hours or shorter) it had persistent activity for up to 7 days for Trichostrongylus and 35 days for Haemonchus, as a result of release from fat depots, so it is a long acting ML oral formulation in sheep. Injections, implants and intra-ruminal capsules and boluses can make these short acting ML's long acting, as a result of paying out for longer.

Preferably both parts are given to the animal at the same time (simultaneously) either as two formulations using a single delivery device (such as dual injection gun), or combined chemically into one formulation (remembering that ML's and levamisole are incompatible and degrade at least in aqueous formulations unless partitioned or held in a non-aqueous formulation) and given by a single delivery device (injection gun).

It should also be recognised that the release profiles required for the ML and levamisole are different with levamisole being released rapidly while the ML is released slowly. This is a technical difficulty and one that is not fully solved by pour ons which give erratic profiles, require 2-2.5 times the quantity of an anthelmintic, and do not give a constant high rapid blood peak for levamisole that it requires to make it highly effective.

Currently there is no injectable levamisole formulation available for use with cattle in New Zealand and only one injectable levamisole formulation in combination with a clostridial vaccine (Nilvax) for sheep. So the combined use of an injectable ML and injectable levamisole is not presently possible in cattle, and while an injectable ML and oral levamisole in cattle is possible it is not usual practice. Only a pour on or injectable formulation is practical for large heavier cattle under normal farm conditions. It is also not usual practice to use a dose level of levamisole in cattle below the generally accepted levels of 7.5 - 8.0 mg / kg bodyweight.

A combination ML (abamectin) and levamisole pour on in cattle has recently been developed (Eclipse, Ancare) and is now marketed for use on cattle in New Zealand. The levamisole profile from pour ons has been shown to be variable and slower releasing (which makes it less effective and more likely to promote the development of resistance to levamisole) and the ML component is not believed to have the period of 14 days persistence of Virbamec LA injection.

It is considered that only rapidly released levamisole such as from an oral drench, an injection (or possibly implant) would give the desired release profile.

Our recent trials of the Virbamec LA injection showed that Virbamec LA injection was highly effective in killing incoming larvae including the early E4 larvae of highly ML resistant adult Cooperia worms for 14 days in young (10-16 weeks) calves at weaning.

However after Virbamec LA injection's direct anthelmintic effect of 14 days there was a rapid reinfection of the calves, followed by a host derived anthelmintic-like reaction that resulted in a highly significant drop in egg count, with a drop in Cooperia E4 larvae and other stages and a weight gain was observed at 35 to 42 days after treatment and was similar to other studies conducted by the applicant. The magnitude of these self-treatment effects were of a size and significance comparable with animals being subjected to an anthelmintic treatment. Calves treated with Virbamec LA injection 49 days previously still showed the benefit of treatment relative to controls even though the anthelmintic effect of Virbamec LA injection had ceased 35 days previously.

The controls had been treated with a highly effective oral combination "Arrest C" 49 days previously. ("Arrest C" produced by Ancare New Zealand Ltd is an oral composition containing albendazole and levamisole for the control of sensitive roundworms that may be resistant to benzimidazole or levamisole).

While oral formulations and even pour ons may trigger the host treatment effect it was noted in our trial that only the calves treated with Arrest C at Day 27 showed the host treatment effect while those control calves (first control group) treated with Arrest C at the start of the trial (Day-49) did not. The egg counts in the first control group monitored weekly over the entire 49 days showed a steady increase over time and no decrease suggestive of self-treatment.

In contrast the calves treated with Virbamec LA injection at Days 49, 42, 35, 28, 21, 14 and 7 all appeared to be responding similarly and even those treated early in the trial days 49 and 42 appeared to show the self-treatment effect. It was considered that the period of 14 days with Virbamec LA injection may be optimal for triggering a host response.

Example 2 - A comparative 49 day faecal egg count study.

A comparative 49 day faecal egg count study and pilot blood pharmokinetic study evaluating the therapeutic and persistent efficacy of an ivermectin (Virbamec LA injection) injection and levamisole injection (or oral) when used simultaneously in combination, relative to a short acting ivermectin (Ivomec injection) injection and levamisole injection (or oral) compared to a combination commercial pour on formulation of abamectin and levamisole in cattle naturally or artificially infected with ML resistant Cooperia worm strains, with the ML and levamisole resistance status confirmed by an ivermectin pour on and levamisole injection (or oral) positive control. Faecal egg count, faecal larval culture, and pilot blood analyte study. Single site, composite infection. Natural and/or artificial infection.

**Objective of the Study -** The aim of this faecal egg count and larval culture study was to evaluate the therapeutic and persistent efficacy resulting from the simultaneous combined use of a persistent ivermectin ("Virbamec LA" injection) and levamisole injection ("Niratil injection", Virbac SA), compared to a short acting ivermectin injection ("Ivomec injection") used simultaneously with a levamisole injection ("Niratil injection", Virbac SA), compared to a commercial combination pour on formulation of abamectin and levamisole ("Eclipse" pour on), and an ivermectin pour on ("Ivomec pour on") alone and a levamisole injection ("Niratil injection") alone relative to an untreated control, in grazing young cattle less than one year of age infected with ML resistant worm strains derived from a composite population of 3 New Zealand farm sites from the North and South Islands over a 63 day period.

## EXPERIMENTAL MATERIALS

### Registered Products

- Ivomec pour-on for cattle and deer - 5mg/ml ivermectin in 2-propanol (623g/litre). Batch NCO1890 exp 6-2008. Registration number: A5303, ACVM Act 1997. Registrant: Merial New Zealand Ltd, Level 3, Merial Building, Osterley Way, Manukau City. Dose 1 ml per 10 kg bodyweight (0.5mg ivermectin/kg).
- Ivomec injection for cattle, sheep and pigs - 10mg/ml ivermectin Batch NF 43510, exp 10-2011. Registration number: A7392, ACVM Act 1997. Registrant: Merial New Zealand Ltd, Level 3, Merial Building, Osterley Way, Manukau City. Dose 1 ml per 50 kg bodyweight (0.2mg ivermectin/kg).
- Eclipse pour-on - 10mg/ml abamectin and 200mg/ml levamisole. Batch 3979, exp 12-2008. Registration number: A9270, ACVM Act 1997. Registrant: Merial New Zealand Ltd, Level 3, Merial Building, Osterley Way, Manukau City. Dose 1 ml per 20 kg bodyweight (0.5mg ivermectin/kg and 10mg/kg levamisole).

## TEST PRODUCTS

Niratil Injectable - 150mg/ml levamisole hydrochloride. Batch no: Lot 1TAD, Expiry 03/10. Registrant: Virbac Santé Animal. Dose rate is 1mm per 20 kg (equivalent to 7.5mg levamisole HCl /kg bodyweight). Administer by subcutaneous or intramuscular injection high in the neck.

- Virbamec LA Injection - 10mg/ml ivermectin. Batch no 36073, Expiry 03/08. Registration number NRA: 55727/1102. Registrant: Virbac (Australia) Pty Limited, 15 Pritchard Place, Peakhurst NSW 2210. Dose rate is 0.02ml/kg or 1ml per 50 kg (equivalent to 0.2mg ivermectin/kg bodyweight), or 2ml up to 100kg. Administer by subcutaneous injection high in the neck behind the ear.

## EXPERIMENTAL DESIGN AND TREATMENT GROUPS:

The 63 day field trial was a controlled faecal egg count (FEC) reduction and larval study in forty-eight (48)) young recently weaned dairy beef (Hereford Friesian) cattle with a mixed worm burden derived from natural infection pasture. The pasture had been deliberately contaminated with a composite population from 3 New Zealand farms with known (<60% FEC reduction) ivermectin (macrocyclic lactone, ML) resistant Cooperia worm strains. The trial farm chosen was initially a "clean" farm with no cattle grazing on the farm for over 10 months (mid Oct 06-Mid Aug 07), and had been used exclusively for hay and grazing horses during this time. Three months prior to the trial commencing nine (9) "seeder" calves that had been treated with a clean out combination drench (Arrest+ Ivomec oral, Oxfendazole+Levamisole+Ivermectin) that were then artificially infected 2 -3 days later with worm strains from 3 farms (2 North Island, 1 South Island) and allowed to contaminate the pasture. Each strain was put into 3 individually identified "seeder" calves (3x3=9) and the egg count was checked at 21-28 days to confirm infection and egg output.

The trial animals were selected from a group of 64 mainly heifer white faced (Hereford x Friesian) 1 to 3 day old calves that were raised on the trial property from birth and kept in the paddocks adjacent to the barn .At weaning they were dosed with a quarantine drench (Arrest+ Ivomec oral, Albendazole+Levamisole+Ivermectin) and then rotationally grazed around the farm paddocks contaminated by the "seeder" calves and exposed to similar natural infective worm challenge by grazing the same pasture as one herd.

Five days prior (Day -5, 2nd Jan 2008) to the dosing day (Day 0, 7th Jan 2008) all 64 calves were individually faecal sampled, weighed (Day -1) then 48 calves were allocated on the basis of this faecal egg count to 6 treatment groups of 8 animals, to give groups with a similar mean and distribution of egg count, and female/male ratio (6 heifers and two steers).

Treatments were as summarised in Table 1 with the eight calves in Group 1 remaining untreated. The calves in Group 2 were treated with a commercial ivermectin pour on alone (Ivomec, positive control), while Group 3 calves were treated with a subcutaneous injectable levamisole formulation alone (Niratil injectable), Group 4 were treated with a commercial abamectin and levamisole pour on formulation (Eclipse pour on), Group 5 calves were treated simultaneously with a short acting ivermectin formulation (Ivomec injection) and a levamisole injection (Niratil injectable) and Group 6 calves treated simultaneously with a long acting ivermectin formulation (Virbamec LA injection) and then a levamisole injection (Niratil injectable). All formulations were applied based on individual body weight (Day -1), to determine the therapeutic efficacy of each formulation at standard label dose rates. The pour on treated animals in Groups 1 and 4 (Ivomec pour on and Eclipse pour on) were treated using plastic syringes with the dose calculated and drawn up to the nearest 0.1ml then applied along the midline of the back, just above or touching the hair, from the withers to tail base. These pour on treated animals were then held separately by individual pour on groups for 3 days after treatment to prevent accidental treatment of another group by licking or rubbing a pour on treated calf. The levamisole and ivermectin injections were applied using 3 ml or 10 ml syringes respectively with a new sterile 20 gauge needle for each injection, and were administered subcutaneously in the right anterior neck just behind the ear. In treatment groups (Groups 5&6) where there was a simultaneous injection applied, the levamisole injection and ivermectin injection was administered immediately adjacent to each other with only a gap of 1-2 cm. Each formulation dosed by a separate syringe given immediately after one another (within 1-2 minutes).

Sampling was as per protocol with bloods collected from the external jugular veins from 3 individually identified animals from Group 4 (Eclipse pour on) and Group 6 (Virbamec LA injection+Levamisole injection) respectively (total 6 calves). Bloods were collected in 10ml plain glass vacutainers at 5 time points (30 minutes, and 1,2,6, and 24 hours after treatment) and were kept cool, allowed to clot, then spun and the sera collected and held frozen (within 24 hours) for the sponsor, in case levamisole testing of sera was required.

Individual faecal samples (for individual egg count and group larval culture) were collected as per protocol at 7 days intervals at Days 7, 14, 21,28,35,42 and 49. A further sampling was performed at Day 63 as part of AHR trial 108 (a pre-trial sampling, for a dose titration of levamisole oral in this ML worm population). As the animals had remained untreated and grazed as one group the results were added in this trial to provide additional information at this time point. Weighing was performed at the beginning, middle, and end of 49 day trial. An additional weighing point at Day 61, part of trial AHR 108 was provided again to add additional information to the report

[Table 1]

Table 1 - Treatment Groups and Group Sizes

| Group | Treatment | Day Treatment | Dose Rates (mg/kg) | Concentration | Dose Volume (mL/kg) | No. Head |
|---|---|---|---|---|---|---|
| 1 | Untreated Control | Nil | Nil | Nil | Nil | 8 |
| 2 | Positive Control Ivomec pour on 14 day withhold | 0 | Ivermectin 0.500 mg/ kg | 5mg/ml | 1ml/10kg | 8 |

(continued)

| Group | Treatment | Day Treatment | Dose Rates (mg/kg) | Concentration | Dose Volume (mL/kg) | No. Head |
|---|---|---|---|---|---|---|
| 3 | Levamisole injectable (Niratil) | 0 | Levamisole 7.5mg/kg | 150mg/m 1 | 1ml/20kg | 8 |
| 4 | Test Product "Eclipse" 35 day withold | 0 | Abermectin 0.500 mg/kg | 10mg/ml | 1ml/20kg | 8 |
| | | | Levamisole 10mg/kg | 200mg/m 1 | | |
| 5 | Ivomec injection | 0 | Ivermectin 0.200mg/kg | 10mg/ml | 1ml/50kg | 8 |
| 6 | Levamisole injectable (Niratil) | 0 | Levamisole 7.5mg/kg | 150mg/m 1 | 1ml/20kg | 8 |
| | TestProduct Virbamec L A injection | | Ivermectin 0.200mg/kg | 10mg/ml | 1ml/50kg | |
| | Levamisole injectable (Niratil) | | Levamisole 7.5mg/kg | 150mg/m 1 | 1ml/20kg | |

All treated calves were observed for two hours after treatment. They were also observed during the 6 and 24 hour bleeding time points for adverse reactions including at application sites. Observations of some immediate swelling in those injected with the levamisole injection (Niratil injectable) at 24 hours after injection resulted in observations for the injection site being monitored at weekly intervals starting from Day 3 instead of Day 7.

## TEST ANIMALS

The forty-eight (48) trial animals were white face dairy and beef cross (Herford-Friesian) calves, with 6 heifers and 2 male steers per group, all with low positive egg counts. The calves were aged approximately 5 months at the beginning of the study and were fully weaned. The calves were selected from a group of 64 calves with a mean body weight of 87.3kg (range 52-109.5 kg) with a mean egg count of 125epg with all but 2 calves having positive egg counts (62 positive, 2 negative counts) The test animals were 64 calves specifically reared for the trial that had been treated with a clean out quarantine drench at weaning and then allowed to graze pasture contaminated by "seeder" calves that had previously contaminated the pasture with worm strains originating from 3 farms with known ML resistant Cooperia. The calves were from a variety of farm sources and were transported to the trial farm from 1-3 days of age and reared on the property. Grass feed calves just after weaning were chosen for the trial because of their greater susceptibility to parasitism and because they are commonly treated at this age with anthelmintics

## STUDY FACILITIES AND EQUIPMENT

The trial farm had not had any cattle grazing for over 8 months previously and had been used for cropping hay and graze horses exclusively. The farm has a gently sloping to flat in contour and is subdivided by 8-wire post and batten fences into 9 paddocks up to 2 acres.

## SOURCE OF INFECTION / INFESTATION

The gastro-intestinal nematode infection was derived from natural field infection, acquired by the trial animals as they grazed infective nematode larvae on pasture. The worm population was however unique as it was a composite worm population derived from 3 New Zealand farms (two from the North Island and one from the South Island) all with a marked ML resistance (<60% reduction) to ivermectin as a pour on or oral. The farm had previously had no cattle for over 10 months and had been used exclusively for making hay and horse grazing. The composite worm pasture population was created by using nine (9) "seeder" calves that were rotationally grazed as one mob 2 months prior to the start of the trial. The "seeder" calves were created by initially treating these calves with a clean out combination drench (Arrest C+ Ivomec oral, Albendazole+Levamisole+Ivermectin) then dosing approximately 2-3 days later with infective larvae (3-10,0001arvae) given over 3 days to each animal (from 9,000-30,000 larvae). The larvae used in this study were from a North Island Dairy farm (Helensville), a South Island Dairy farm (Canterbury), and a South Auckland beef farmer (Pukekohe). Three individually identified calves were dosed with the larvae originating from one of the 3 farms (3 x3=9 seeder calves) creating an initial pure strain from one of these 3 farms with infection confirmed by positive egg count 21-28 days later.

## ALLOCATION TO TREATMENT

At the start of the trial Day -5, sixty-four (64) individually tagged and identified calves were faecal sampled and then weighed using calibrated scales (Day -1) and their sex, breed and age also recorded.

The 64 calves were ranked by egg count from highest to lowest. Starting from the highest counts the calves were then blocked into 8 blocks of 6 calves with the lowest egg count or lightest body weight or clinically abnormal calves excluded. The calves in each block were allocated at random by drawing numbers from a hat to one of the 6 treatment groups. This gave 6 treatment groups (8 calves per group) with a similar mean and distribution of egg count. The sex ratio to give 6 heifer and 2 steers per group was achieved in the final allocation by swapping a female and male calf of identical egg count between 2 groups.

## PRE-TREATMENT OBSERVATIONS

Of the 64 calves reared for the trial all but three calves appeared clinically normal. The three calves that were excluded from selection from the trial were one which had a lymphadenitis and was stunted (Tag 722), one which had a ringworm lesion on the midline of the back (Tag 731) and one which had a hernia (Tag 781)

## ADMINISTRATION OF TREATMENTS

The ivermectin injections (Virbamec LA injection and Ivomec injection) were given by subcutaneous injection to the nearest 0.1ml using a 3ml plastic syringe (syringe marked in 0.1ml graduations). Niratil was given by a 10ml syringe also to the nearest 0.1ml. The dose was administered by holding the animal standing, tenting the skin around the base of the right ear, injecting downwards and delivering the entire dose volume using a clean 20 gauge x 3/8 inch metal Luer lock needle just beneath the skin at a single injection site as per protocol and label, and in the trial photos.

Pour-on application for both Eclipse pour on and Ivomec pour on was by way of a plastic 10ml syringe with individual dose rates calculated by live weight to the nearest 0.1ml and the product applied against the skin and hair, along the midline of the back from the withers to the tail base and is also shown in the trial photos.

Oral dosing of larvae in the "seeder" calves was by pipetting a measured volume dose (ml) of liquid larval culture (of known genera and concentration) onto filter paper (Whatman No.1 filter paper , 10cm diameter) then folding and rolling this up while slightly damp into a ball and dosing the damp ball over the back of the tongue using a sheep "Extender" capsule dosing gun. This method is one that had been used extensively by Lincoln University and prevents any spitting out of liquid and larvae that can occur if a liquid culture is dosed (Robin McAnulty, personal communication).

## DOSE RATES

Dose rates were applied at standard dose rates as listed in Table 2.

Ivomec pour on alone was applied to Group 2 calves at 1 ml per 10kg based on individual body weight (to nearest 0.1ml) as per protocol, to give an ivermectin dose rate of 0.500mg /kg body weight.

Niratil injectable (levamisole hydrochloride) was given by subcutaneous injection at a dose rate of 1ml per 20kg (to nearest 0.1ml) to give a dose rate of 7.5mg/kg, alone in Group 3 and in combination with injectable ivermectin formulations in Groups 5 & 6.

Eclipse pour on (abamectin+levamisole) was applied at a dose rate of 1ml per 20kg to give a dose rate of 0.500mg/kg abamectin and 10mg/kg levamisole.

Ivomec injection (a short acting ivermectin injection) was given subcutaneously at a rate of 1ml per 50kg equivalent to a dose of 0.200mg/kg to Group 5 animals while those in Group 6 were dosed with Virbamec LA injection (an ivermectin injection with more persistent activity) was dosed at a similar rate of 1ml per 50kg equivalent to 0.200mg/kg ivermectin.

[Table 2]

Table 2 - Treatment Groups and Dose Rates used (mg/kg body weight)

| Product | Group | Dose | Levamisole (mg/kg) | Abamection (mg/kg) | Ivermectin (mg/kg) |
|---|---|---|---|---|---|
| Nil | 1 Control | Nil | Nil | Nil | Nil |
| Ivomec pour on | 2 | 1ml/10kg | Nil | Nil | 0.500mg/kg |
| Naratil injectable | 3 | 1ml/20kg | 7.5mg/kg | | |
| Eclipse pour on | 4 | 1ml/20kg | 10mg/kg | 0.500mg/k g | |
| Ivomec injection + Niratil injectable | 5 | 1ml/50kg + 1ml/20kg | 7.5mg/kg | | 0.500mg/kg |
| Virbamec LA injection + Niratil injectable | 6 | 1ml/50kg + 1ml/20kg | 7.5mg/kg | | 0.500mg/kg |

## CONCURRENT MEDICATION

The calves were treated with Niltime LV (Virbac NZ Ltd Batch 23441 A, Exp 06-2008, Bendiocarb 40g/l) for lice during the trial. The calves had all been dosed with a clean out Arrest (Albendazole+Levamisole) and Ivomec oral (ivermectin) over one month prior to the commencement of the study then allowed to naturally infect with a composite worm population off pasture. No other medication was given.

## SAMPLES TAKEN

Blood for serum levamisole analysis were collected from 3 identified animals in Groups 4 (Eclipse pour on) and 5 (Virbamec LA injection +Niratil injectable) as per protocol. The blood sample was collected in 10ml plain glass vacutainers from the external jugular vein at 5 time points (30 minutes, and 1,2,6, and 24 hours after treatment). The blood samples and were kept cool, allowed to clot, and the sera was spun and held frozen (within 24 hours).

Individual faecal samples were collected from the rectum as per protocol at 7 day intervals at Days 7, 14, 21,28,35,42 and 49. A further sampling was performed at Day 63 as part of AHR trial 108 (a pre-trial sampling). Each animal was faecal sampled separately using a clean glove to prevent any cross contamination. The individual faecal egg count was performed to sensitivity 50epg. A pooled composite larval culture (25g) was performed on each treatment group over the duration of the trial.

Weighing was performed using calibrated scales at the beginning, middle, and end of 49 day trial. An additional weighing point at Day 61, part of trial AHR 108 is also provided to add additional information to the report

## LABORATORY TEST METHODS

Faecal egg counts were performed using a modified McMaster technique to a sensitivity of 50 epg, with larval culture recovered by Baermann technique and stained with iodine and identified under 100 x magnifications.

## POST-TREATMENT OBSERVATIONS

General clinical observations on the safety of all trial formulations including the injectable and pour on products, including at the site of injection and at the pour on site were made at 7 time points as per protocol.

While all pour ons and injectable formulations were well tolerated at the time of application, a subcutaneous fluid swelling at the site of Niratil (levamisole hydrochloride) injection was noted in some animals at 24 hours post treatment in all 3 groups (Groups 3,5,6) that had been injected with this product and the trial Sponsor and its monitor immediately notified. The planned examination time point at Day 7 in the protocol was move forward to Day 3 to determine the extent of the swelling (including grading the size and severity of the reaction) with the assessment by both the study director/investigator and the study monitor provided in this report.

## CALCULATIONS AND STATISTICAL ANALYSIS

To assess treatment effect data was entered into a computer Microsoft Excel spreadsheet. Individual faecal egg count and strongyle worm counts were then collated by treatment groups and the arithmetic means calculated using the spreadsheet. The anthelmintic efficacy (percentage reductions) for each group were determined for both post treatment faecal egg and worm stages relative to those in the untreated controls (Group 1) using arithmetic means and were calculated as follows;

[Table 3]

$$\% \text{ Efficacy} = \frac{(\text{mean count in control group} - \text{mean count in treated group}) \times 100}{\text{Mean count in control group}}$$

The individual body weights over the trial period, and also at Day 61 (part of AHR trial 0108) were similarly entered into the Microsoft EXCEL spreadsheet. This data was then collated by group and the difference in mean body weight at different time periods through the trial was calculated including over the entire 61 days.

**KEY STUDY DATES**

[Table 4]

Table 3 - Trial Activities

| Trial Day | Activity |
|---|---|
| | Dose "Seeder" calves with clean out Arrest C+Ivomec oral |
| | Start dosing Seeder calves with 5-10,000 larvae/day |
| | Finish dosing Seeder calves with 5-10,000 larvae/day |
| | Monitor faecal egg count in seeder calves (all positive) |
| Day -5 | Individually tag& faecal sample (using clean glove per calf) 64 calves & record sex & breed |
| Day -1 | Individually weight calves and record weights. Allocate to group. Calculate dose volumes/rates per individual weight |
| Day 0 | Identify calf to group (tag and colour code). Treat all 6 groups as per protocol. Remove Control group before treatments given. Treat pour on treatments last and keep them separate and apart from other pour on group and other treatments. Identify and blood sample 3 calves each in Group 4 (Eclipse) and Group 6 (Virbamec LA plus Niratil injection), total 6 calves at 30min, 1,2, and 6 hours. Observe all treated animals for any adverse effect. |
| Day 1 | Bleed 6 calves, 3 calves each in Group4 and 6 (24 hour time point) |
| Day 3 | Yard all calves. Observe/palpate all calves at injection & pour on sites, including both sides of neck |
| | Observe measure and record any swelling in the region of the injection site for all calves including those in the injectable groups, Groups 3, 5&6. |
| Day 7 | Collect faeces (All Groups, 48 calves) for FEC and pooled group larval culture (6). Observe pour on site |
| Day 10 | Observe measure and record any lesion in region of the injection site for all calves including those in the injectable groups, Groups 3, 5&6. |
| Day 14 | Collect faeces (All Groups, 48 calves) for FEC and pooled group larval culture (6). Observe pour on site |
| Day 17 | Observe measure and record any lesion in region of the injection site for all calves including those in the injectable groups, Groups 3, 5&6. |
| Day 21 | Collect faeces (All Groups , 48 calves) for FEC and pooled group larval culture (6), Observe pour on site |
| Day 24 | Observe measure and record any lesion in region of the injection site, Groups 1, 5&6. |
| Day 28 | Collect faeces (All Groups, 48 calves) for FEC and pooled group larval culture (6). Observe pour on site .Weigh all trial animals & record |

(continued)

| Trial Day | Activity |
|---|---|
| Day 31 | Observe measure and record any lesion in region of the injection site, Groups 3, 5&6. |
| Day 35 | Collect faeces (All Groups, 48 calves) for FEC and pooled group larval culture (6). Observe pour on site |
| Day 39 | Observe measure and record any lesion in region of the injection site, Groups 3, 5&6. |
| Day 42 | Collect faeces (All Groups, 48 calves) for FEC and pooled group larval culture (6). Observe pour on site. Weigh all trial animals & record |
| Day 46 | Observe measure and record any lesion in region of the injection site, Groups 3, 5&6. |
| Day 49 | Collect faeces (All Groups, 48 calves) for FEC and pooled group larval culture (6). Observe pour on site |
| Day 61 | Weigh all trial animals & record (Part of AHR108) |
| Day 63 | Collect faeces (All Groups, 48 calves) for FEC and pooled group larval culture (6). (Part of AHR108) |

**Best Mode for Carrying out the Invention**

**RESULTS-BACKGROUND DISCUSSION**

All trial products including the registered commercial formulations of Ivomec pour on (Ivermectin), Ivomec injection and Eclipse pour on (Ivermectin+Levamisole), and the test injectable products of Niratil injectable (levamisole hydrochloride) injection and Virbamec LA injection were all dosed based on individual body weight at the standard label dose rates. These products were dosed to weaned calves with positive egg counts that were known to be infected with a composite worm population derived from 3 properties from the North and South Island that had confirmed ivermectin (ML) resistant Cooperia worm strains.

In this current study calves were treated with ivermectin pour on alone (Group 2), and compared with those treated with levamisole injection alone (Niratil, Group 3), or those treated with ML +levamisole combinations including a commercially available pour on (Eclipse, abamectin+levamisole, Group 4)) or simultaneous combination injections with the 2 injections (ivermectin +levamisole) given immediately adjacent to each other. Two different ivermectin injections of different durations of activity were tested consisting of the short acting ivermectin injection (Ivomec inj+Niratil inj, ivemectin+levamisole, Group 5) and a slightly longer acting ivermectin injection (Virbamec LA inj+Niratil inj, ivermectin + levamisole, Group 6).

The trial was designed to monitor egg counts and larval cultures at weekly intervals for 49 days after treatment and an additional time point was added at Day 63.

The trial calves had remained untreated and grazed as one group until this time. It was anticipated from observations and scientific literature that Ivomec pour on would remove virtually all worm species except adult or immature resistant Cooperia, while levamisole injection alone (Niratil injectable) would remove virtually all Cooperia including ML resistant strains, and the ML+ levamisole combinations would remove virtually all worms. However based on previous worm count data there was evidence in another Cooperia strain that while the adult and L4 stage may be highly resistant to ML's such as ivermectin, that the E4 stage appeared fully ML sensitive. It was therefore speculated that this trial design using ML+ Levamisole combinations in a composite ML resistant Cooperia population, originating from wide geographic locations would show if this observation was a general rule for New Zealand Cooperia strains. Additionally if this were true then this trial design would also be expected to demonstrate the different persistent activity periods of the combination formulations, as all resistant adult and L4 Cooperia stages would be removed by levamisole and the persistent activity periods of the different ML formulations would be expressed because of the on-going kill of the E4 stages. Even ML resistant Cooperia worm strains would be prevented from developing into the resistant L4 and adult stages during this persistent activity period. Cooperia eggs would only return in these ML +levamisole combination treated calves when the ML persistent periods had ceased and E4 stages could re-establish and complete their development to patent egg laying adult stages.

The trial design was also expected to demonstrate if the apparent self-treatment effect observed in the previous Virbamec LA study, which included a drop in egg count is a common feature in young calves.

Group mean data is presented in the following report with more detailed individual data by group shown in the summary tables at the end of the report.

The trial was conducted in midsummer in drought conditions in south Auckland. The temperatures varied from minimum temperatures as low 10.8°C, with maximum air temperatures up to 32.2° C, and most days of 22-28° C. There was virtually no rainfall with the only rain consisting of light misty showers with approximate 20mm rain/ month (See Daily Log and Meteorological Data in Appendices). There was no rain within 6 hours of pour on application.

**PARASITOLOGY & BODYWEIGHTS**

**Faecal Egg Counts**

Prior to the trial commencing all trial calves were orally dosed with a levamisole and albendazole combination anthelmintic drench known as Arrest C (this is the trade name of a product from Merial Ancare New Zealand Limited, Osterley Way, Manukau, Auckland. A7290. Batch No3054, exp 05/2010.200gm/litre albendazole & 75gm/litre levamisole HCI. Standard dose 1ml per 10kg (10mg/kg albendazole and 7.5 mg/kg levamisole HCI) plus Ivomec oral at standard oral dose rates as clean out dose, and then grazed and allowed to infect naturally on pasture freshly contaminated by "seeder" calves infected with 3 different ML resistant strains from different New Zealand locations. All faecal samples over the entire study were collected by a new clean glove per calf to prevent cross contamination, with faecal sampling weekly from treatment Day (Day 0 to Day 49) with one additional point at Day 63.

At Day -5 faecal sampling used for trial animal selection the 60 trial calves had low but uniformly positive egg counts (range 100-350epg) with the mean counts in the 6 treatment groups varying from 132-150epg. By treatment day (Day 0) the egg counts rose marginally (means 125-213 epg) and for the untreated controls the mean egg count remained at approximately that level until Day 28 to 63 where the counts were higher with mean counts varying from 400 to 525epg throughout this period. All control animals had uniformly positive counts at all samplings over the entire trial, with individual counts varying from 100 to 1000epg in the later part of the study (Days 28-63).

The mean egg count in Ivomec pour on treated calves (Group 2) dropped to their lowest point at Day 7 post treatment with a mean count of 69epg (range 0-150egp, with 2 zero counts) before rising by 14 days after treatment to 119 epg (all positive) and then spiking to 438 epg at 28 days after treatment followed by a decline to 218epg (Day 35) and then a slow rise from Day 35 (213epg) to Day 63 (463epg).

The egg counts in calves treated with Niratil injectable alone (levamisole injection) were all zero at 7 days after treatment (mean 0 epg) with very occasional low positive counts (50 epg) detected at 14 and 21 days after treatment (mean egg counts 13epg and 6 epg respectively), before slowly rising with uniformly positive counts at Day 28 (mean 181epg) before slowly rising to 263epg then falling marginally to 219epg by Day 63.

The egg counts for both Eclipse pour on (abamectin+levamisole) treated calves in Group 4 and those treated with Virbamec LA injection and Niratil injection (Ivermectin+Levamisole, Group 6) showed a very similar trend. In both groups the egg counts remained zero (mean= 0 epg) for 28 days after treatment with positive counts only detected at Day 35. In the Eclipse pour on treated group the egg counts were marginally lower than those in the Virbamec LA + Niratil treated group (Group 6) for Days 35 to 63, with the egg count steadily rising from 19 epg at Day 35 to 131 epg, 169 epg and 182 epg at Days 42,49, and 63 respectively. By contrast the egg counts in calves treated with Virbamec LA injection +Niratil injection (Group 6) showed a more rapid rise in egg count of 63 epg at Day 35, rising to 244 epg at Days 42 and 49, then declining to 225 epg at Day 63.

The egg counts for those treated with Ivomec injection and Niratil injection (Group 5) showed a similar pattern of egg count to those treated with Virbamec LA and Niratil injection (Group 6) except zero counts were only observed up to 21 days after treatment (instead of Day 28 for Group 6, Virbamec LA +Niratil) with a low count (mean 50 epg) at Day 28 followed by a rise in egg count of 113 epg (Day 35) to 288 epg at Day 42 before the egg count declined to 262 epg and then 250epg at Days 49 and 63 respectively.

[Table 5]

Table 4 - Arithmetic mean (AM) faecal egg counts over the trial. Treatment Day (Day 0)

| Treatment Group | Trial Day | | | | | | | | | |
|---|---|---|---|---|---|---|---|---|---|---|
| | -5 | 0 | 7 | 14 | 21 | 28 | 35 | 42 | 49 | 63 |
| | Egg Count (EPG) | | | | | | | | | |
| Group 1 Control | 138 | 194 | 256 | 156 | 281 | 400 | 525 | 413 | 369 | 519 |
| Group 2 Ivermectin Pour On | 150 | 125 | 69 | 119 | 94 | 438 | 218 | 289 | 300 | 463 |
| Group 3 Levamisole | 150 | 194 | 0 | 13 | 6 | 181 | 213 | 263 | 256 | 219 |
| Group 4 Abamectin+ Levamisole Pour on | 150 | 213 | 0 | 0 | 0 | 0 | 19 | 131 | 169 | 182 |
| Group 5 Ivermectin +Levamisole Injection | 138 | 194 | 0 | 0 | 0 | 50 | 113 | 288 | 262 | 250 |
| Ivermectin LA + Levamisole Injection | 132 | 132 | 0 | 0 | 0 | 0 | 63 | 244 | 244 | 225 |

**Faecal Egg Count Reductions (%) of Treated Groups**

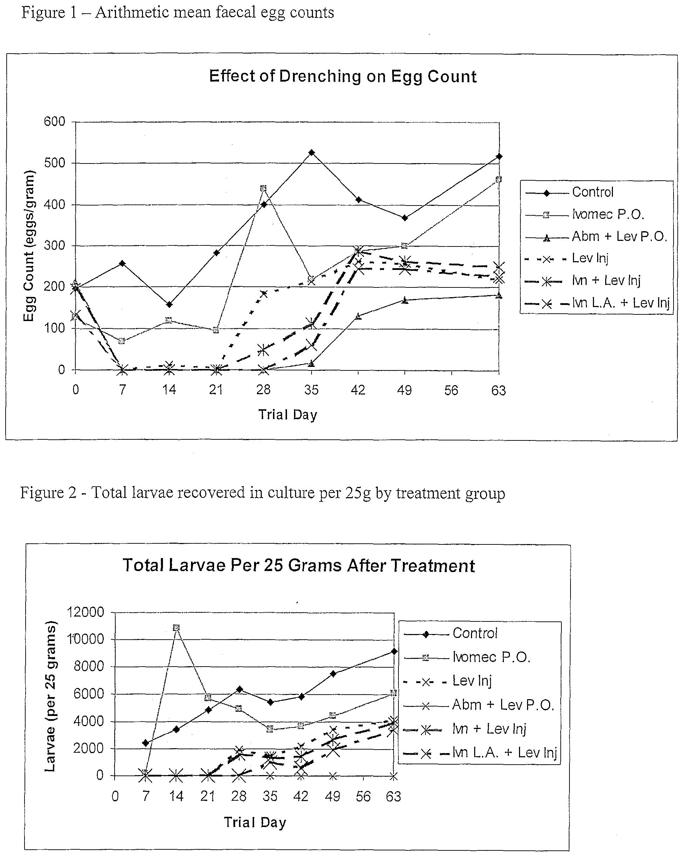

The faecal egg count reductions (%) for each treatment group, based on arithmetic means of each group relative to the untreated controls (Group 1) are shown in Table 5 up to 63 day s after treatment. The pattern of efficacy as determined by egg count reduction (%) generally reflected the pattern of the raw mean egg counts for each group. There were two distinct patterns depending on treatment type. In those groups not receiving levamisole (the controls and also ivermectin pour on, Groups 1&2) the egg counts rose over the later part of the trial, while in those groups treated with levamisole (Groups 3-6) the egg counts initially rose after treatment (and the efficacy declined) but the egg counts then dropped (and the efficacy rose) over the later part of the trial. This can be seen graphically in Figure 1 showing group mean egg count.

As in the summary efficacy table (Table 5) the groups treated with products with persistent activity can be seen with complete reductions (100%) in egg count for 28 days in Groups 4 and 6 (Eclipse pour on, and Virbamec LA injection+ Niratil injectable respectively), with a slightly shorter period of 21 days for Group 5 (Ivomec injection+Niratil injectable). Niratil injectable itself (Group 3) gave only one time with a complete reduction in egg count (Day 7) and for Ivomec pour on there was no time point where a complete reduction was observed. Observations for each group were as follows:

For Ivomec pour on (ivermectin pour on, Group 2) there was an incomplete reduction with a 73 % reduction 7 days after treatment and only a 24 % reduction relative to controls 14 days after treatment confirming Cooperia worm strains (confirmed by larval culture) with adult stages highly resistant to ivermectin. At Day 21 the reduction was higher at 67% but by 28 days after treatment there was no reduction in egg count with counts higher than the controls (-9%). The efficacy relative to controls then increased to 58% at 35 days after dosing, then slowly reduced from 30, to 19% and then to 11% at 42, 49 and 63 days after treatment respectively.

In contrast Niratil gave a complete reduction in egg count (100%) by day 7 after treatment and maintained relatively efficacy from day 14 (89%) to 21 (93%) before declining on Day 28 (59%) to only 3% efficacy (similar to controls) 35 days after treatment, before efficacy increased from 9% (Day 42), then 15% (Day 49) to 53 % at Day 63 after treatment.

In the Eclipse (abamectin+levamisole) pour on treated calves, a complete reduction in egg count (100% efficacy) was observed for the first 28 days , and was still high at 91% at Day 35, then declined to moderate levels of 44-61% at Days 42 to 61%. In the combined Ivomec and Niratil injection group 100% efficacy was seen to progressively decline to zero (no difference to controls) at Day 42 and then rise to 13 % at Day 49 and even higher at Day 63 (46%). The same pattern was seen with combined Virbamec LA and Niratil injection with 100% efficacy seen for one week longer to Day 28 with a progressive reduction to 15% of controls at Day 42 before progressive rising at Day 49 to 19% then rising further to 51 % at Day 63.

The results show a slow sinusoidal pattern of faecal egg count counts and egg count reductions (%) over time following treatment in calves.

[Table 6]

Table 5 - Efficacy (%) by treatment group. Percentage (%) reduction in arithmetic egg count versus untreated controls.

| Treatment Group | Trial Day | | | | | | | |
|---|---|---|---|---|---|---|---|---|
| | 7 | 14 | 21 | 28 | 35 | 42 | 49 | 63 |
| | Efficacy. Percentage reduction (%) vs Untreated Controls | | | | | | | |
| Group 2 Ivomec PO (Ivn) | 73 | 24 | 67 | -9 | 58 | 30 | 19 | 11 |
| Group 3 Niratil Inj (Lev) | *100* | 89 | 93 | 59 | *3* | *9* | 15 | 53 |
| Group 4 Eclipse PO (Abm + Lev) | *100* | *100* | *100* | *100* | 54 | 54 | *44* | 61 |
| Group 5 Ivomec Inj + Niratil Inl (Ivn + Lev) | *100* | *100* | *100* | 89 | *0* | *0* | 13 | 46 |
| Group 5 Virbamec LA + Niratil Inj (Ivn + Lev) | *100* | *100* | *100* | *100* | *15* | *15* | 19 | 51 |

Italic figures showing when reductions are highly effective and when the reductions are at their lowest for that group

**Faecal Larval Cultures**

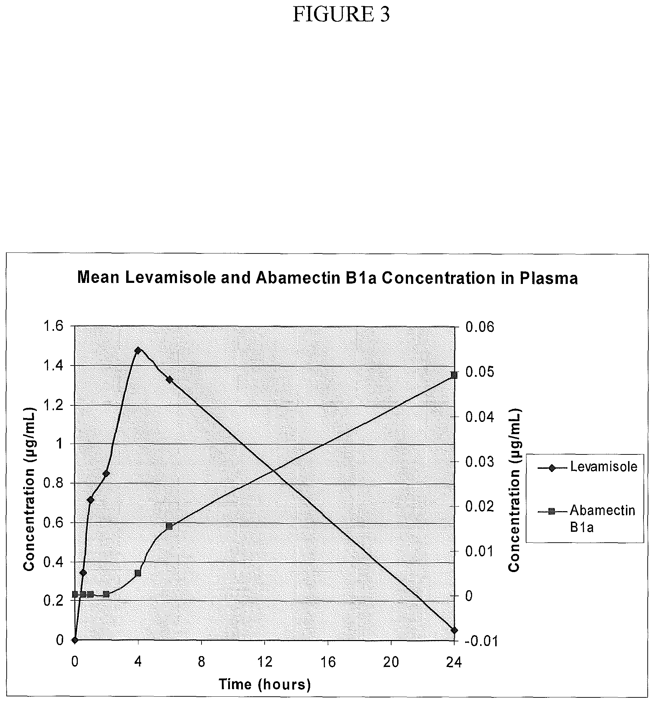

The pooled faecal larval culture results of each of the treatment groups (Groups 2-6) and the controls (Group 1) are shown by group in separate Tables (Tables 6-11) and the total larvae recovered per 25 grams of pooled faeces from each group over time is shown in Figure 3. All larvae in culture were differentiated by worm genera type expressed as a percentage (%), even though in some larval cultures post treatment contained only one or two larvae, hence the percentage figures need to be interpreted in light of the number of larvae recovered as presented below. While total number of larvae recovered generally does follow the egg count, this is not always true as describe below (see Group 2 (Ivomec pour on) below).

The larval cultures for the untreated controls showed a gradual rise in larval numbers recovered in culture over the trial period with 2400 larvae/25g at Day 0 rising to 9200 larvae/25g by Day 63. The larval composition was relatively stable over the entire 63 day period consisting predominantly of Cooperia larvae varying from 88-98%, with Ostertagia larvae also consistently present at low numbers (2-9%), small numbers of Haemonchus larvae usually but not always detected (up to 4%) and very occasional Trichostrongylus (up to 2%) detected at two time points at Day 0 and Day 63 and Oesophagostomum/Chabertia (up to 1%) at Day 7 and 42.

There were several overall trends observed in larval cultures. Only in levamisole treated groups (Groups 3-6) were there time points where no larvae were recovered.

Larvae (Cooperia) were consistently recovered from Group 2 the Ivomec pour on treated animals often in very high numbers soon after treatment (10800 larvae/25g at Day 14) the highest larval recovery in the entire trial despite an egg count at this time of 119epg.

For Cooperia species larvae significant numbers (>100 larvae/25g) were present at all times following treatment with Ivomec pour on (Group 2), but were not detected until Day 28 following treatment in Groups 3 and 5 (Niratil injection alone or in combination with Ivomec injection), or until Day 35 for Group 6 (Virbamec LA + Niratil injectable) or until Day 42 for Eclipse pour on. Allowing a 21 day prepatent period this would give protection period of 7, 14 and 21 days respectively for these groups.

For Ostertagia species larvae did not return in significant numbers (>100 larvae/25g) until Day 49 for Ivomec pour on (Group2), which was the same as that observed for Eclipse pour on (Group 4).

For Niratil injectable and Ivomec Injection+ Niratil injectable (Groups 3&5) Ostertagia larvae in significant numbers were detected by Day 28 but were not detected until Day 35 in those treated with Virbamec LA injection+Niratil injectable (Group 6). Based on these observations and a prepatent of 21 days a protection (persistent activity) period for Niratil injectable alone and Ivomec Injection+Niratil would be 7 days, for Virbamec LA+Niratil it would be 14 days and for Ivomec pour on and Eclipse pour on it would be 28 days.

Because of the low numbers and intermittent nature of detection of Haemonchus, Trichostrongylus and Oesophagostomum/Chabertia larvae no accurate protection period could be determined.

However no Haemonchus larvae were found following treatment following ML treatment (Groups 2,4,5,6) but were detected in the group treated with Niratil alone (Group 3) at Day 35.

[Table 7]

Table 6 - Worm genera composition and total larval numbers/g in the faeces from untreated controls (Group 1) over the trial (Day 0 to Day 63)

| Group 1 | | | | | | | | | |
|---|---|---|---|---|---|---|---|---|---|
| Control | 0 | 7 | 14 | 21 | 28 | 35 | 42 | 49 | 63 |
| **Worm Genera** | | | | | | | | | |
| Haemoncus (%) | 1 | 1 | 4 | 0 | 0 | 1 | 0 | 0 | 4 |
| Ostertagia (%) | 6 | 2 | 8 | 3 | 9 | 5 | 7 | 2 | 6 |
| Tichonstrongylus (%) | 2 | 0 | 0 | 0 | 9 | 0 | 0 | 2 | 2 |
| Cooperia (%) | 91 | 96 | 88 | 88 | 91 | 94 | 92 | 98 | 88 |
| Oespho/Chabertia | 0 | 1 | 0 | 9 | 0 | 0 | 1 | 0 | 0 |
| | | | | | | | | | |
| Larvae per 25 gram | NQ | 2400 | 3400 | 4800 | 6300 | 5400 | 5800 | 7500 | 9200 |

[Table 8]

Table 7 - Worm genera composition and total larval numbers/g in the faeces from Group 2 over the trial (Day 0 to Day 63). Ivomec pour on (ivermectin) treated at Day 0

| Group 2 | | | | | | | | | |
|---|---|---|---|---|---|---|---|---|---|
| Ivomec pour on | 0 | 7 | 14 | 21 | 28 | 35 | 42 | 49 | 63 |
| **Worm Genera** | | | | | | | | | |
| Haemoncus (%) | 1 | 0 | 0 | 0 | 0 | 0 | 0 | 0 | 0 |
| Ostertagia (%) | 6 | 0 | 0 | 0 | 0 | 0 | 0 | 3 | 9 |
| Tichonstrongylus (%) | 2 | 1 | 0 | 0 | 0 | 0 | 0 | 0 | 2 |
| Cooperia (%) | 91 | 99 | 100 | 100 | 100 | 100 | 100 | 97 | 91 |
| Oespho/Chabertia | 0 | 0 | 0 | 0 | 0 | 0 | 0 | 0 | 0 |
| | | | | | | | | | |
| Larvae per 25 gram | NQ | 130 | 1080 0 | 5700 | 4900 | 3400 | 3700 | 4400 | 6100 |

[Table 9]

Table 8 - Worm genera composition and total larval numbers/g in the faeces from Group 3 over the trial (Day 0 to Day 63). Niratil injectable (levamisole) treated at Day 0

| Group 3 | | | | | | | | | |
|---|---|---|---|---|---|---|---|---|---|
| Niratil inj | 0 | 7 | 14 | 21 | 28 | 35 | 42 | 49 | 63 |
| **Worm Genera** | | | | | | | | | |
| Haemoncus (%) | 1 | 0 | 0 | 0 | 0 | 1 | 0 | 0 | 0 |
| Ostertagia (%) | 6 | 0 | 0 | 100 | 11 | 0 | 9 | 5 | 11 |
| Tichonstrongylus (%) | 2 | 0 | 4 | 0 | 0 | 0 | 0 | 0 | 0 |
| Cooperia (%) | 91 | 0 | 96 | 0 | 89 | 99 | 91 | 95 | 89 |
| Oespho/Chabertia | 0 | 0 | 0 | 0 | 0 | 0 | 0 | 0 | 0 |
| | | | | | | | | | |
| Larvae per 25 gram | NQ | 0 | 25 | 15 | 1950 | 1480 | 2200 | 3400 | 4200 |

[Table 10]

Table 9 - Worm genera composition and total larval numbers/g in the faeces from Group 4 over the trial (Day 0 to Day 63). Eclipse pour on treated (Abamectin+Levamisole) at Day 0

| Group 1 | | | | | | | | | |
|---|---|---|---|---|---|---|---|---|---|
| Group 4 | 0 | 7 | 14 | 21 | 28 | 35 | 42 | 49 | 63 |
| **Worm Genera** | | | | | | | | | |
| Haemoncus (%) | 1 | 0 | 0 | 0 | 0 | 0 | 0 | 0 | 0 |
| Ostertagia (%) | 6 | 0 | 0 | 0 | 0 | 0 | 0 | 11 | 7 |
| Tichonstrongylus (%) | 2 | 0 | 0 | 0 | 0 | 0 | 0 | 0 | 0 |
| Cooperia (%) | 91 | 0 | 0 | 0 | 100 | 100 | 100 | 89 | 93 |
| Oespho/Chabertia | 0 | 0 | 0 | 0 | 0 | 0 | 0 | 0 | 0 |
| | | | | | | | | | |

(continued)

| Group 1 | | | | | | | | | |
|---|---|---|---|---|---|---|---|---|---|
| Larvae per 25 gram | NQ | 0 | 0 | 0 | 4 | 12 | 230 | 1640 | 2900 |

[Table 11]

Table 10 - Worm genera composition and total larval numbers/g in the faeces from Group 5 over the trial (Day 0 to Day 63). Ivomec Injection (ivermectin) and Niratil injectable (levamisole) treated at Day 0

| Group 5 | | | | | | | | | |
|---|---|---|---|---|---|---|---|---|---|
| Control | 0 | 7 | 14 | 21 | 28 | 35 | 42 | 49 | 63 |
| **Worm Genera** | | | | | | | | | |
| Haemoncus (%) | 1 | 0 | 0 | 0 | 0 | 0 | 0 | 0 | 0 |
| Ostertagia (%) | 6 | 0 | 0 | 0 | 5 | 0 | 0 | 2 | 12 |
| Tichonstrongylus (%) | 2 | 0 | 0 | 0 | 0 | 0 | 0 | 0 | 0 |
| Cooperia (%) | 91 | 0 | 100 | 0 | 95 | 100 | 100 | 98 | 88 |
| Oespho/Chabertia | 0 | 0 | 0 | 0 | 0 | 0 | 0 | 0 | 0 |
| | | | | | | | | | |
| Larvae per 25 gram | NQ | 0 | 1 | 0 | 1600 | 1300 | 1400 | 2700 | 3900 |

[Table 12]

Table 11 - Worm genera composition and total larval numbers/g in the faeces from Group 6 over the trial (Day 0 to Day 63). Virbamec LA injection (ivermectin) and Niratil injectable (levamisole) treated at Day 0

| Group 6 | | | | | | | | | |
|---|---|---|---|---|---|---|---|---|---|
| Control | 0 | 7 | 14 | 21 | 28 | 35 | 42 | 49 | 63 |
| **Worm Genera** | | | | | | | | | |
| Haemoncus (%) | 1 | 0 | 0 | 0 | 0 | 0 | 0 | 0 | 0 |
| Ostertagia (%) | 6 | 0 | 0 | 100 | 0 | 0 | 7 | 7 | 6 |
| Tichonstrongylus (%) | 2 | 0 | 0 | 0 | 0 | 0 | 0 | 0 | 0 |
| Cooperia (%) | 91 | 0 | 0 | 0 | 100 | 100 | 96 | 93 | 94 |
| Oespho/Chabertia | 0 | 0 | 0 | 0 | 0 | 0 | 1 | 0 | 0 |
| | | | | | | | | | |
| Larvae per 25 gram | NQ | 0 | 0 | 1 | 25 | 990 | 600 | 1900 | 3400 |

**Body Weights**

Individual body weights of all the trial calves were recorded at the beginning, middle and end of the Virbac study with an addition time point added at Day 61. Throughout this 61 days the calves remained grazing as one group, apart from the two pour on groups kept apart from all other groups including the other pour on group for the first 72 hours immediately after treatment to prevent any licking of pour on by any other groups. The animals were not treated with any other product apart from Niltime (bendiocarb) for lice, a treatment approved by the sponsor and a product that was not considered to compromise the trial results in any respect. Although not allocated by weight to treatment groups, all 6 groups were relatively similar in mean body weight varying from 86.4 to 92.8kg at the start of the study. In the first 29 days of the study from Day -1 to Day 28 the mean body weight gain in the untreated controls (Group 1) was 10.3 kg, with Ivomec pour on (ineffective against Cooperia), Niratil injectable (not fully effective against Ostertagia and little persistent activity) or Eclipse pour on (which appeared fully effective against all worm genera) having lower weight gains of 9.1kg, 9.4kg and 9.1kg respectively. In contrast the two groups treated with an ivermectin injection and levamisole injection had higher weight gains than the controls over this 28 day period with those treated with Ivomec injection and Niratil injectable (Group 5) gaining an average of 11.3kg and those treated with Virbamec LA injection and Niratil injectable (Group 6) gaining 11.7kg.

In the following 14 days (Day 28 to Day 42) the highest weight gain was 5.2kg in Group 4 (Eclipse pour on) followed by Virbamec LA+Niratil treated animals (Group 6, 5.0kg), then Ivomec pour on treated animals (Group 4, 4.2kg), followed by the Ivomec injection+Niratil treated group (Group 5, 3.8kg) the controls (Group 1, 3.6kg) and the Niratil injectable only treated group (Group 3, 3.4kg). In the last 19 days from Day 42 to Day 61 the picture was somewhat similar with Group 3 treated with Niratil alone gaining the least weight at 2.6kg, while the controls gained 3.1 kg, followed by Eclipse pour on treated animals (Group 4, 3.2kg), then the Ivomec Injection and Niratil treated group (Group 5, 3.4kg), with Ivomec pour on treated animals gaining 4.6kg (Group 2) and those treated with Virbamec LA injection+Niratil (Group 6) gaining the most weight of 4.7kg over this time period.

Over the entire 63 day period the Virbamec LA injection +Niratil injectable group (Group 6) gained the most weight of 21.4kg, followed by the Ivomec injection+Niratil injectable group (Group 5, 18.4kg), then the Ivomec pour treated animals (Group 2, 17.9kg) the Eclipse treated group (Group 4, 17.8kg), then the untreated controls (Group 1, 17kg) with the lowest weight gain in the Niratil alone treated group of 15.4kg (Group 3). While the group sizes were small (8 per group) the observation that the Eclipse treated group grew slower than controls in the first 28 days following treatment but then grew at rates faster than controls and similar to the ML+levamisole injectable combinations, is consistent with previous observations in New Zealand growth response trials for single levamisole pour ons in particular the pioneer commercial product Anthelpor or Levipor (A 04197, 200g/l levamisole). A previous New Zealand comparative growth response trial conducted by the author also showed cattle treated with the pioneer levamisole pour on growing slower than the controls while those treated with an injectable ML (doramectin) and ML pour on (moxidectin) grew significantly faster (Bomatak pour on dossier A 07479, Nov 1996). In this study it is also interesting to note that in the group treated with a short acting levamisole injection (Niratil injectable), which did not fully control adult Ostertagia or have significant persistent activity against incoming Ostertagia or Cooperia had consistently lower growth rates than the controls over the entire trial, while those anthelmintics that did control adult Ostertagia and had persistent activity against incoming Ostertagia and Cooperia larvae (including Ivomec pour on) all gained more weight over the trial than the controls.

[Table 13]

Table 12 - Mean Body Weight of treatment groups at the start, middle and end of trial with weight gains for the first 29 days (Gain A), the second 14 days (Gain B) and the last 19 days (Gain C) as well as total gain over the entire 63 day period relative to controls (Group 1).

| Group | Wt (kg) | Wt (kg) | Wt Gain (kg) | Wt (kg) | Wt (kg) | Wt Gain B (kg) | Wt (kg) | Wt(kg ) | Wt Gain (kg) | Total Wt Gain |
|---|---|---|---|---|---|---|---|---|---|---|
| | | | 29 days | | | 14 days | | | 19 days | 63 days |
| Day (D) Date | D-16 Feb | D284 Feb | Diff | D284 Feb | D 42 18 Feb | Diff | D 42 18 Feb | D 61 18 Mar | Diff | Total (kg) |
| Group 1 Contro 1 | 86.5 | 96.8 | 10.3 | 96.8 | 100.4 | 3.6 | 100.4 | 103.5 | 3.1 | 17.0 |
| Group 2 Ivomec PO (Ivn) | 90.0 | 99.1 | 9.1 | 99.1 | 103.3 | 4.2 | 103.3 | 107.9 | 4.6 | 17.9 |
| Group 3 Niratil Inj (Lev) | 87.6 | 97.0 | 9.4 | 97.0 | 100.4 | 3.4 | 100.4 | 102.9 | 2.6 | 15.4 |
| Group 4 Eclips ePO (Abm + Lev) | 87.5 | 96.6 | 9.1 | 96.6 | 102.1 | 5.4 | 102.1 | 105.3 | 3.2 | 17.8 |
| Group 5 Ivomec Inj + Niratil Inj (Ivn + Lev) | 92.8 | 104.1 | 11.3 | 104.1 | 107.9 | 3.8 | 107.9 | 111.3 | 3.4 | 18.4 |

(continued)

| Group | Wt (kg) | Wt (kg) | Wt Gain (kg) | Wt (kg) | Wt (kg) | Wt Gain B (kg) | Wt (kg) | Wt(kg ) | Wt Gain (kg) | Total Wt Gain |
|---|---|---|---|---|---|---|---|---|---|---|
| Group 6 Virbamec LA Inj + Niratil Inj (Ivn + Lev) | 86.4 | 98.1 | 11.7 | 98.1 | 103.1 | 5.0 | 103.1 | 107.8 | 4.7 | 21.4 |

[Table 14]

Table 13 - Egg and larval contamination estimate per calf for the untreated controls (Group 1), Ivomec pour on (Group 2) and those treated with Virbamec LA injection +Niration Injectable. Based on a 100kg calf producing 5 kg faeces per day and using the arithmetic mean egg count or total larvae/g for the first 21 days (Days 7 to 28) of the second 28 days (Days 35 to 63) of the trial, with a pro rata calculation for the second 21 days(Day 35 to 56)

| Average FEC or larvae/g (D7-D28) | Weight of Faeces in 21 days per calf (Day 28-7) | Total Contamination per Calf in 21 days (eggs/larvae) (D7-D28) | Average FEC or larvae/g (D35-D63) | Weight of faeces in 28 days (Day 63-35) | Total Contamination per Calf in 21 days (eggs/larvae) (D 35 to 56) (pro rata) | Total Contamination per Calf in 28 days (eggs/larvae) (D35 to 63) |
|---|---|---|---|---|---|---|
| Control. Egg Contamination (1st 21 days and 2nd 28 and 21 days (pro rata)) | | | | | | |
| 257.5 epg | 105,000g | 27,037,500 | 456.3epg | 140,000g | 47,907,560 | 63,876,750 |
| Ivomec pour on. Egg contamination (1st 21 days and 2nd 28 and 21 days (pro rata)) | | | | | | |
| 161.3 epg | 105,000g | 16,931,250 | 317.2epg | 140,000g | 33,304,690 | 44,406,250 |
| Virbamec LA inj+Niratil inj. Egg contamination (1st 21 days and 2nd 28 and 21 days (pro rata)) | | | | | | |
| 0 epg | 105,000g | 0 | 193.8epg | 140,000g | 20,343,750 | 27,125,000 |
| Control. Larval Contamination (1st 21 days and 2nd 28 and 21 days (pro rata)) | | | | | | |
| 169 larvae | 105,000g | 17,745,000 | 279 larvae | 140,000g | 29,295,000 | 39,060,000 |
| Ivomec pour on. Larval contamination (1st 21 days and 2nd 28 and 21 days (pro rata) ) | | | | | | |
| 215 larvae | 105,000g | 22,606,500 | 176 larvae | 140,000g | 18,480,000 | 24,640,000 |
| Virbamec LA inj+Niratil inj. Larval contamination (1st 21 days and 2nd 28 and 21 days (pro rata)) | | | | | | |
| 6.5 larvae | 105,000g | 27,300 | 68.9larvae | 140,000g | 7,234,500 | 9,646,000 |
| | | | | | | |

Day 0 excluded as pre-treatment value, start at first sampling post treatment (Day 7)
Faecal Output per 100kg calf for 21 days= 5kg/day x 21 days=105kg or 105000g
Faecal Output per 100kg calf for 28 days= 5kg/day x 28 days=140kg or 140000g

**GENERAL DISCUSSION**

This faecal egg count and larval culture study provides further confirmation and support for the findings and observations in Example 1.
As planned in the protocol the worm population derived from three farms with highly ML resistant Cooperia worms strains (2 from South and North Auckland and 1 from Canterbury, including 2 strains from dairy farms) resulted in a composite and highly ML resistant Cooperia worm population. Ivomec pour on treatment in this study (Group 2) resulting in only a 24% percentage reduction in egg count relative to controls at 14 days post treatment with a larval culture at this time point where 10,800 larvae/25 g were recovered, and a larval composition that was 100% Cooperia. This larval recovery was the highest in the entire trial including the untreated controls.
As in the background discussion, one of the purposes of the study was to confirm the observation of Example 1 that despite highly resistant adult and egg producing Cooperia stages, the immature stages and particularly the E4 stage larvae stages appeared still fully ML sensitive. Based on this observation it was theorised if this was a general rule for New Zealand ML resistant Cooperia strains, then removal of ML resistant Cooperia and in particular the adult and late L4 stages with levamisole would result in the different persistent activity lengths of ML drenches being observed both in egg count and larval culture. The reason is that the life cycle of even ML resistant Cooperia life would be disrupted. Only when the ML susceptible E4 stages could re-establish at the end of the ML persistent activity period could development then continue through to the egg producing adult stages and eggs return in faeces. Based on New Zealand trials this period for Cooperia is almost exactly 21 days (treatment to time of egg production in non-persistent drenches), and this period is very similar to Ostertagia. Using a composite population from widely different locations as in this trial was considered a simple way of testing if this was in fact a common or general rule. If it were not true, and the incoming and E4 stages were in fact ML resistant then these resistant Cooperia larvae would continue to establish and the persistence periods (often 14 days or longer with some ML formulations) would not be seen, so egg production would occur much earlier at approximately 21 days post treatment.

In this study the different persistent periods of different ML formulations are clearly seen in those groups treated with levamisole, which has removed all the ML resistant Cooperia stages. Only in Ivomec oral treated calves that were not treated with levamisole (Group 2) is the persistent activity period against the susceptible Cooperia E4 larvae masked. This is due to the surviving ML resistant Cooperia adults continuing to produce eggs after treatment. In this trial the egg counts of Ivomec pour on treated calves even exceed those of the untreated controls by Day 28.

In contrast those calves treated with Virbamec LA injection in combination with levamisole (Niratil injectable) demonstrated a full protection period (100% efficacy) for 28 days with a small numbers of Ostertagia and Cooperia returning 35 days after treatment supporting a 14 day persistent activity period against incoming and E4 larvae for these worm genera (14 days persistent activity+21 days to patency=35 days to egg production). This 14 day persistent activity period is consistent with worm count data from Example 1 which used Virbamec LA injection alone.

Based on the egg count reductions (%) and larval cultures in this current trial the calculations of persistent efficacy against incoming/E4 larvae for Ivomec injection was 7 days for both Cooperia and Ostertagia, while for Eclipse pour on it was 21 days persistent activity for Cooperia and 28 days for Ostertagia.

While the persistent activity against incoming and E4 Cooperia larvae could not be established for Ivomec pour on as the animals had not been treated with levamisole, the recovery of Ostertagia larvae at day 49 or longer suggests that like Eclipse, Ivomec pour on has a similar 28 day persistent activity period against this worm genera (28 day persistent activity+ 21 day prepatent period=49 days)

Niratil injection alone appeared to have a persistent activity period against both Ostertagia and Cooperia of 7 days or less.

Although occasional Haemonchus larvae were detected in the controls and also in the Niratil injectable only treatment (Group 3) at Day 35, no persistent period could be determined for the ML formulations. Equally however no Haemonchus were detected in any ML treated group for the entire 63 days of the trial.

One of the other objectives of the study was to observe if there was evidence of the same unexplained " anthelmintic like" reduction in egg count that occurred soon after the return and rise in egg count after treatment as seen in Example 1. This "anthelmintic like" action was speculated to be most probably a host response to limit particularly Cooperia infection or egg output after treatment. In this present trial also conducted in young calves just after weaning, but in a different season and worm population, this same reduction in egg count was again observed and is most graphically illustrated in Figure 1. As shown in Figure 1 in all treatments receiving levamisole injection (Groups 3, 5 & 6) there is an initial rise in egg count following treatment and reinfection, but this rise does not continue but instead the egg count falls between Days 49 and 63 while those of the controls, Ivomec pour on and Eclipse pour on actually continue to rise over this time. These findings support the view that this appears as a real effect and has implications not only in identifying a previously unknown self-treatment effect following some forms of anthelmintic treatment but also has implications for retreatment interval and also lowering the contamination of eggs and therefore larvae on pasture.

While the group sizes in this study were small (8 per group) and animals were not allocated on the basis of weight, the trends in this trial as in the previous Example 1 appears significant. Despite the treatments Eclipse pour on (Group 4) and Virbamec LA injection+ Niratil Injectable (Group 6) both giving apparently very good worm control (with Eclipse pour on having at least 1-2 weeks more persistent activity against both Ostertagia and Cooperia than Virbamec LA injection), this pour on combination did not result in higher weight gains than the injectable combination but in fact the reverse. The major reason for this observation was in the first 28 days the Eclipse pour on treated group actually gained less weight than the controls, while those groups treated with the injectables including Virbamec LA injection+Niratil injectable (Group 6) and those treated with Ivomec injection+ Niratil injectable (Group 5) actually grew faster than the controls. This weight gain observation following Levamisole pour on treatment in calves has been seen previously by the author in other comparative weight gain trials and it is considered that it may also be a real effect. A possible cause would seem to be that there is a negative growth effect soon after levamisole pour on treatment that overrides the initial benefit of greater worm control. It is speculated it may be the solvents or the way levamisole is presented to the animal.

It is also noted in this study that even the levamisole injection (Niratil) alone treated group also grew slower than controls during this period. This negative effect on growth is further illustrated by the observation that those treated with Ivomec pour on actually gained more weight than Eclipse pour on treated calves, despite Ivomec pour on not removing the adult ML resistant Cooperia stages. The weight gains from highest to lowest over the 63 day period were Virbamec LA injection+Niratil (21.4kg), Ivomec injection+Niratil (18.4kg), Ivomec pour on (17.9kg), Eclipse pour on (17.8kg), Untreated controls (17kg) and Niratil alone (15.4kg). This difference in weight gain and also body condition could be seen visually and is shown in the trial photos.

Table 13 shows that the combination regime of this invention is more effective than the comparison treatments. In this trial very high level of pasture contamination from ML resistant adult Cooperia occurred immediately following treatment at levels approximating that of untreated animals and would result in a rapid shift in the worm population on pasture to the resistant genotype. Assuming each 100kg calf produces 5kg of faeces using both the egg count and the total number of larvae recovered data (adjusted to 1g), then the output of eggs or larvae (in brackets) per calf from Day 7 to Day 28 of the first 21 days of the trial is 27,037,500 (17,745,500), 16,931,250 (22,606,500) and 0 (27,300 larvae) for one untreated control, Ivomec pour on, or Virbamec LA injection+ Niratil (Levamisole) injection treated calf respectively in this trial. As such the contamination rate for Ivomec pour on is a similar magnitude as the untreated controls with vast numbers of eggs originating from ML resistant Cooperia adults being deposited on pasture. In contrast there was virtually no contamination of pasture from calves treated with Virbamec LA injection in combination with levamisole.

In the second 28 days of the trial from Day 35 to 63 when Cooperia (including ML sensitive) and Ostertagia had already re-established in the Virbamec LA injection+levamisole group (Group 6) the egg output over this period for a 100kg calf from this group is 27,125,00(9,646,000 larvae) which is similar in magnitude to those of Ivomec pour on and the untreated controls in the first 21 days. The corresponding counts at that time (last 28 days) for these groups were at least 50% higher for Ivomec pour on (44,406,250 eggs, 24,640,00 larvae), or 100% higher in the case of controls (63,876,750 eggs,39,060,00 larvae). As such the effect of Ivomec pour on alone treatment was not to reduce contamination but to shift the pasture contamination to ML resistant worm strains in the first 28 days. In complete contrast the effect of Virbamec LA injection +levamisole was to virtually eliminate all pasture contamination (including from the ML resistant Cooperia worm strains) in the first 21 days then allow both susceptible and resistant Cooperia to equally reinfect in the last 28 days, but at a reduced pasture contamination level, and includes an effect which appears to be a secondary "host derived" effect on egg output. These observations suggest that there is no shift or obvious selection advantage favouring the ML resistant worm with this injectable combination, but instead a huge benefit in decreasing pasture contamination and the need for retreatment. The other issue highlighted in this trial is that with ML pour ons it is almost impossible to determine an appropriate retreatment interval. If it is done prior to Day 49, such as regular 4-6 week retreatment intervals then Ostertagia with either Ivomec pour on or Eclipse pour on would have insufficient time to become patent and re-establish on pasture. With the injectable combinations such as with Virbamec LA + levamisole the shorter persistency period of 14 days, means Ostertagia larvae and ML sensitive Cooperia are re-establishing on pasture at 35 days post treatment and the re treatment interval could be 5-6 weeks or in reality considerably longer without applying selection pressure for either Ostertagia or Cooperia to develop resistance to complete their life cycle.

## STUDY CONCLUSIONS

This New Zealand field study in 5 month old weaned calves using a composite worm population derived from 3 widely distributed New Zealand cattle farms with highly resistant ivermectin (ML) resistant worm strains further confirms that Virbamec LA injection has a persistent activity period of 14 days against incoming or E4 Ostertagia larvae, and also 14 days activity against incoming or E4 Cooperia larvae. As in the previous Virbac Trial 010605 it appears that the E4 stage of these Cooperia strains has remained fully ML sensitive even in New Zealand Cooperia populations with highly ML resistant adult stages.

The simultaneous injection of Virbamec LA injection (ivermectin) and Niratil injectable (levamisole) to remove ML resistant Cooperia resulted in the expression of the persistent activity period with complete reductions (100%) in egg count for 4 weeks (28 days), and also the highest weight gains of any combination product tested including Eclipse pour on (abamectin +levamisole).

Although not presented in this study a levamisole dose titration study conducted later on the ML resistant Cooperia strains (AHR 0108) in these calves following this present study confirmed that these strains were still highly levamisole sensitive with doses of ¼ to 1/3rd lower than the standard label dose (7.5mg/kg) giving full (100%) control.

Additionally there is evidence in this trial that the ML pour ons such as Eclipse pour on and Ivomec pour on have very long periods of selective persistent activity against Ostertagia with patency with this genera only evident at 49 days after treatment. This creates issues for retreatment intervals. Although Cooperia egg counts may return earlier, frequent repeated treatment intervals of 4 -6 weeks (28-42 days) for a ML pour on would appear to apply selection pressure to Ostertagia species to become ML resistant in order to complete its life cycle.

The apparent lack of growth response following Eclipse pour on application despite effective worm control is also of concern. One possible explanation if this effect is real is that there may be a negative effect on growth from levamisole pour on use, and that this is masking the positive effect of effective worm control.

In contrast the ML+ levamisole injectable combination, particularly Virbamec LA injection + levamisole appears to provide a more sustainable, effective and superior worm control tool than current pour on formulations, and one which may also utilise a temporary self-treatment effect from the host including on egg output. In this trial Virbamec LA injection+ levamisole appeared highly effective in preventing any significant pasture contamination for 4 weeks after treatment. Unlike the ML pour ons in this study it appears Virbamec LA injection has a persistent period of only 14 days after which ML susceptible Ostertagia and Cooperia larvae are able to establish with infections of both genera patent and detected in larval culture 35 days after treatment. As such in combination with levamisole, Virbamec LA injection appears non selective for ML resistant Cooperia (including incoming larvae) from 14 days post treatment. Even with short treatment intervals such as 5-6 weeks susceptible Cooperia and particularly Ostertagia still have time to complete their life cycle and so this is less likely to be strongly selective for resistant genotypes. Additionally it also appears there may also be a temporary host reaction to limit further Cooperia infection as a result of injectable treatment which could further extend retreatment intervals and reduce drench use.

[Table 15]

Table 14 - Summary table comparing the faecal egg and larval contamination of untreated controls, Ivomec pour on alone and Virbamec LA injection +Niratil injectable. Based on a 100kg calf producing 5 kg faeces per day and using the average egg count or total larvae/g per group for the first 21 days (Days 7 to 28) of the second 28 days (Days 35 to 63) of the trial.

| FIRST 21 DAYS (DAY 7 to DAY 28) | | SECOND 28 DAYS (DAY 35 to DAY 63) | |
|---|---|---|---|
| EGGS | LARVAE | EGGS | LARVAE |
| Total Contamination per Calf in 21 days (eggs) (D7 -D28) | Total Contamination per Calf in 21 days (larvae) (D7 -D28) | Total Contamination per Calf in 28 days (eggs/larvae) (D35 to 63) | Total Contamination per Calf in 28 days (eggs/larvae) (D35 to 63) |
| GROUP 1. CONTROL | | | |
| 27,037,500 | 17,745,000 | 47,907,560 | 39,060,000 |
| GROUP 2. IVOMEC POUR ON | | | |
| 16,931,250 | 22,606,500 | 33,304,690 | 24,640,000 |
| GROUP 3. VIRBAMEC LA INJECTION+NIRATIL INJECTABLE | | | |
| 0 | 27,300 | 20,343,750 | 9,646,000 |

Faecal Output per 100kg calf for 21 days= 5kg/day x 21 days=105kg or 105000g

Faecal Output per 100kg calf for 28 days= 5kg/day x 28 days=140kg or 140000g

Day 0 excluded as pre-treatment value, start at first sampling post treatment (Day 7)

Whilst the method of this invention has been proven to be effective using two different formulations, it is far preferable to include the two different actives in a single formulation. We have found that it is possible to make stable injectable formulations, containing both actives, and the following Examples 3 - 6 describe suitable formulations.

Our most preferred formulation is that of Example 3 which describes a combined injectable anthelmintic formulation containing both abamectin and levamisole base.

In making these formulations we found the levamisole base was more effective than levamisole hydrochloride, or levamisole phosphate, although Example 5 gives an Example containing levamisole phosphate. We have found the levamisole base is far more stable in these injectable formulations, and provides minimal irritation when injected into an animal.

EXAMPLE 3 - describes a combined injectable anthelmintic formulation:

Abamectin 1.00%
Levamisole base 37.50%
Butylhydroxyanisole 0.02%
Butylhydroxytoluene 0.01%

Glycerol formal (stabilized) qs to 100 mL
* Specific Gravity is approximately 1.21
The following examples show other injectable anthelmintic formulations.

EXAMPLE 4

Eprinomectin 1.00%
Levamisole Base 40.0%
Butylhydroxyanisole 0.02%
Butylhydroxytoluene 0.01%
Glycerol formal (stabilized) qs to 100 mL

EXAMPLE 5

Ivermectin 1.00%
Levamisole phosphate 50.0%
Butylhydroxyanisole 0.02%
Butylhydroxytoluene 0.01%
NMP * qs to 100 mL
(NMP is the standard abbreviation for N-Methyl-2-pyrrolidone)

EXAMPLE 6

Moxidectin 1.00%
Levamisole Base 30.0%
Butylhydroxyanisole 0.02%
Butylhydroxytoluene 0.01%
Glycerol formal (stabilized) qs to 100 mL

EXAMPLE 7

A controlled field trial in young calves evaluating the safety of a new combination abamectin and levamisole injection as per Example 1 referred to herein as "CombatAbaLev" at label (1x) using the formulation example. Animals were dosed at the standard dose rate 1x (0.2mg/kg abamectin, 7.5mg/kg), 2x (twice) and 3x (three times) that dose rate". This includes study of the local safety (tolerance) at the injection side compared to sterile water or saline for injection (within the same animal), and relative to a registered abamectin injection, including a comparative pilot efficacy (faecal egg count reduction/larval cultures at the label dose rates (1x). Target Animal Safety study. Pilot Efficacy"

Study design. Twenty-nine dairy beef cross 8-10 week old calves of mixed sex were individually identified with a unique ear tag then weighed and faecal sampled for roundworm egg count. The calves were naturally infected and originated from a north Waikato calf rearing unit, with the calves having a mean body weight of 85kg (65-114kg) at the start of the trial. Calves were fed whole cow's milk once a day until approximately Day 20 then weaned. Twenty five calves were allocated to group by body weight to give 3 groups with similar mean weight consisting of Group 1a (5 calves), Group 2 (10 calves, Combat AbaLev) and Group 3 (10 calves, Abamectin injection), with the remaining 4 calves allocated to the controls (Group 1b) to provide additional efficacy (FEC and larval culture data). The calves were noted to have quite severe ringworm which was graded prior to the start of the trial, animals with more severe neck lesions were allocated to the controls. The small number of female calves were allocate to the control (Group 1, 2 females) and test product (Group 2, 4 females), with Group 3 consisting entirely of bull calves.

The study was conducted as both a safety and pilot efficacy study. In the first part lasting 27 days, the 10 calves in Group 2 were injected subcutaneously with Combat AbaLev at standard label dose rate (1x) and the 10 calves in Group 3 were injected with Genesis Injection at the standard label dose rate (1x). The injected animals also received a similar volume of saline or water injection on the opposite side of the neck to compare reactions with the injectable anthelmintic products.

The injection sites of those receiving Combat AbaLev were compared with those of Genesis injection. The temperatures, respiration and heart rates, blood parameters and general observations of Combat AbaLev treated calves were compared with pre-treatment levels and also 5 control calves to determine safety. Body weights, observations at the injection site and faecal samples were also taken over the first 27 days and presented below. Blood (plasma) was also collected from 3 animals treated with Combat AbaLev in the first 24 hours in order to establish a blood profile.

After the first 27 days the calves in Groups 2 and 3 were mixed and reallocated to two new groups of 10 animals each (2 b and 2c). These two groups were injected with Combat AbaLev at double the standard dose rate (2x) either as a single dose (Group 2b) or as a split dose (Group 2c, two 1x doses). As in first part of the study the treated animals were monitored against 5 untreated controls including temperatures, respiration and heart rates, blood parameters and general observations and the injection sites and body weights were also recorded.

It was noted that the ringworm in the Combat AbaLev treated animals appeared to be resolving faster in the treated animals so observations at the end of the trial was performed to evaluate this effect.

The results are presented below:

Faecal Egg Count and Larval Culture Results
As shown in Table 15

Combat AbaLev was highly effective giving complete (100%) reduction in worm egg counts over 27 days of the trial. In contrast Abamectin injection was found to be ineffective by Day 17 (60% reduction) and Day 27 (25%).

The larval cultures confirmed that even at Day 7 Genesis Injection was not effective at reducing Cooperia larval numbers in faeces, with 560 larva/50g recovered versus 1800/50g (a 66 % reduction). In contrast Combat AbaLev was highly effective at all time points including Day 27, with no larvae recovered. It was found the FEC test was insensitive at the standard diagnostic rate of 50 epg giving a false result, while the more sensitive test of 25 epg correctly estimated that Genesis injection was ineffective (see Day 17 data).

These observations suggest Combat AbaLev has at least 7 days persistent activity to give reductions of this magnitude as Cooperia is able to and readily establishes and is egg laying by 20- 21 days (20+7). It also supports the view that Combat AbaLev would result in very little pasture contamination after treatment and the retreatment interval will also be very long, very likely in the order of 8-10 weeks. With the high degree of contamination with ML resistant worm strains Genesis injection will require more frequent treatment despite its persistence claims.

[Table 16]

TABLE 15 - Faecal Egg Count (Arithmetic Mean) & Efficacy (% reduction)

| Group | Day 7 | Day 17 | Day 17 | Day 27 |
|---|---|---|---|---|
| Test Sensitivity | (50egp) | (50epg) | (25 epg) | (25 epg) |
| Group 1. Controls | | | | |
| FEC (EPG) | 62.5 | 125 | 125 | 116.7 |
| | | | | |
| Group 2. Combat AbaLev Injection. (Abamectin+ Levamisole) | | | | |
| FEC (EPG) | 0 | 0 | 0 | 0 |
| Efficacy (AM) | 100% | 100% | 100% | 100% |
| Group 3. Genesis Injection. (Abamectin) | | | | |
| FEC (EPG) | 0 | 0 | 50 | 85 |
| Efficacy (AM) | 100% | 100% | 60% | 25% |

[Table 17]

TABLE 16 - Faecal Larval Culture Results after treatment

| | Total larvae/ 50g | Heamonchus | Ostertagia | Trichs | Cooperia |
|---|---|---|---|---|---|
| Day 7 | | | | | |
| Controls | 1800 | 0 | 9 | 0 | 91 |

(continued)

|  | Total larvae/ 50g | Heamonchus | Ostertagia | Trichs | Cooperia |
|---|---|---|---|---|---|
| CombatAbaL ev | 6 | 0 | 0 | 0 | 100 |
| Genesis inj | 560 | 0 | 0 | 0 | 100 |
| Day 17 | | | | | |
| Controls | 2100 | 0 | 18 | 0 | 82 |
| CombatAbaL ev | 0 | 0 | 0 | 0 | 0 |
| Genesis inj | 2840 | 0 | 0 | 0 | 100 |
| Day 27 | | | | | |
| Controls | 2100 | 0 | 24 | 0 | 76 |
| CombatAbaL ev | 0 | 0 | 0 | 0 | 0 |
| Genesis inj | 2700 | 0 | 0 | 0 | 100 |

Body Weights and Weight Gain

The body weight results in the first 26 days shows that Combat AbaLev grew faster than controls but slightly less than those with Genesis injection, although those in the Genesis injection group were all bull calves that would be expected to have slightly higher growth rates. It should also be remembered that Genesis injection was not fully effective against Cooperia while Combat AbaLev was.

Of particular interest however is that the calves treated with Combat AbaLev while growing slower in the first 22 days actually grew relatively faster in the later part of the trial from Day 22 to Day 26, relative to Group 3. The Genesis injection group. This observation makes the investigator suspect just as was observed in previous trials that with levamisole treatment (as either a pour on or injection) that there may be some initial and short term suppression of growth. It is speculated that this may be related to levamisole action in the host, quite possible the result of immune stimulation/ modulation (which may divert energy/protein synthesis to the immune system and away from growth) or even increases in interferon levels which are known to depress growth. This mechanism is interesting in view of observations as it would suggest Levamisole may be supporting or assisting host to mount an immune response possible also to gastrointestinal worms. It may also be a factor in why Combat AbaLev treated calves have better controlled ringworm infection in this trial, as it is know that immune suppressed or poor calves are more prone to infection and severity of infection. Levamisole is claimed to restore immune function

[Table 18]

TABLE 17 - Mean Body Weight (Kg)

|  | Day -2 | Day 22 | Day 26 | Day 52 |
|---|---|---|---|---|
|  | Weight (kg) | Weight (kg) | Weight (kg) | Weight (kg) |
| Group 1 Controls | | | | |
|  | 79.72 | 81.70 | 86.72 | 97.30 |
| Group 2. Combat AbaLev. 1x at Day 0 and 2x after Day 27 | | | | |
|  | 87.95 | 93.3 | 101.75 | 115.2 |
| Group 3. Genesis injection .1x at Day 0 then 2x Combat AbaLev after Day 27 | | | | |
|  | 87.05 | 94.40 | 101.50 | 117.80 |

[Table 19]

TABLE 18 - Mean Body Weight Gain (Kg)

| | Weight Gain (kg) Day-2 to Day 22 | Weight Gain (kg) Day 22 to Day 26 | Weight Gain (kg) Day -2 to Day 26 | Weight Gain (kg) Day 26 to Day 53 | Weight Gain (kg) Day-2 to Day 53 |
|---|---|---|---|---|---|
| Group 1 Controls | | | | | |
| | 1.9 | 5.1 | 7 | 10.6 | 17.6 |
| Group 2. Combat AbaLev. 1 x at Day 0 and 2x after Day 27 | | | | | |
| | 5.4 | 8.5 | 13.8 | 13.4 | 27.2 |
| Group 3. Genesis Injection. 1x at Day 0 and 2x Combat AbaLev after Day 27 | | | | | |
| | 7.4 | 7.1 | 14.5 | 16.3 | 30.8 |

Ringworm lesions and effect of treatment

The ringworm scores show that ringworm was relatively evenly spread through the treatment groups at the start of the trial. At the end of the trial those treated with Combat AbaLev had very reduced sized lesions and in some animals they had resolved completely. Skin scrapings and cultures confirm that ringworm is still present even in Combat AbaLev animals. It appears that the host is better able to control and resolve both the infection and lesions. Interestingly those treated with oral levamisole from Day 32 did not resolve or reduce these lesions by Day 59 and in fact the severity in a number of animals actually increased. It is speculated that the form of levamisole (such as injection) and the persistent activity of the anthelmintic in Combat AbaLev may be needed to significantly boost the animals' immunity to control and resolve the ringworm infection. The calves had all been washed with an iodine wash registered for use for ringworm at Day -2 but this was ineffective.

[Table 20]

TABLE 19 - Ringworm scores at beginning and end of trial

| | Day -2 | Day -2 | Day 59 | Day 59 | Day 59 |
|---|---|---|---|---|---|
| | Head lesions | Neck lesions | Head lesions | Neck lesions | Largest Head lesion (cm) |
| Group 1. Controls. Treated after Day 32 with Arrest (Albendazole+levamisole) oral | | | | | |
| Mean severity score | 0.8 | 0.4 | 1.6 | 0.7 | 8.7 Range (7-15cm) |
| No of group with lesions | 5 of 9 | 4 of 9 | 7 of 9 | 6 of 9 | 7 of 9 |
| Group 2 | | | | | |
| Mean severity score | 0.8 | 0.4 | 0.4 | 0.1 | 0.9 Range (1-3cm) |
| No of group with lesions | 5 of 10 | 4 of 10 | 4 of 10 | 1 of 10 | 4 of 10 |
| Group 3 | | | | | |
| Mean severity score | 0.6 | 0.3 | 0.35 | 0 | 3.5 Range (3.5) |
| No of group with lesions | 5 of 10 | 3 of 10 | 1 of 10 | 0 of 10 | 1 of 10 |

Reactions at the injection site

At standard dose rates (1x) Combat AbaLev there was only 1 of 10 animals were there was any immediate swelling (24 hours) seen and this was similar to Genesis injection where two animals were initially observed with swelling. This swelling was mild and resolved quickly. It was also noted that one other animal 3006 developed swelling approximately 7 days after injection near the injection site but this too resolved quickly and there was no lesions detected in any animal other than one animal injected into the epidermis not the subcutis in error.

At 2x the dose there was more extensive swelling in 4 of 10 animals (with a single 2x injection) and in 8 of 10 that were injected with two 1 x doses, again these resolved quite quickly, most could only be detected by palpation and were unapparent by eye within 24 -28 days. The swelling was non painful and as in the 1x dose a small numb of animals also developed a small lump at the injection site some 7 days later which resolved in 2-3weeks. This occurred even in animals where there was initially no reaction to the injection for the first 6 days. It is considered by the investigator that this is most likely a cellular response quite possibly triggered by levamisole and was not observed with Genesis Injection (abamectin alone). It is suggested that this time frame would fit with levamisole's stimulation of the cellular immune system and their migration (lyphocytes/monocytes), and it is interesting to note that all single active levamisole formulations including levamisole phosphate and levamisole hydrochloride carry a precautionary statement that injection site reactions have been observed, and it seems possible that it may be in part related to the active levamisole rather than just a reaction to non-active excipients. This may also suggest that levamisole may be more active or immune stimulating when injected rather than by the oral route (see effect of oral levamisole on Ringworm versus Combat AbaLev)

[Table 21]

Table 20 - Reactions at the subcutaneous injection site - Combat AbaLev and Genesis Injection at standard 1x dose rates in Right neck

|  | Left Neck | Right Neck | Left Neck | Right Neck |
|---|---|---|---|---|
|  | Day 1 | Day 1 | Day 28 | Day 28 |
| Group 2 Combat AbaLev Injection. 1x | | | | |
| 3013 | 0 | 0 | 0 | 0 |
| 3016 | 0 | 0 | 0 | 0 |
| 3018 | 0 | 0* | 0 | 0* |
| 3019 | 0 | 4x2 | 0 | 0 |
| 3021 | 0 | 0 | 0 | 0 |
| 3024 | 0 | 0 | 0 | 0 |
| 3031 | 0 | 0 | 0 | 0 |
| 3036 | 0 | 0 | 0 | 0 |
| 3039 | 0 | 0 | 0 | 0 |
| 3046 | 0 | 0 | 0 | 0 |
| Group 3 Genesis Injection 1x | | | | |
| 3011 | 0 | 0 | 0 | 0 |
| 3014 | 0 | 1x5 | 0 | 0 |
| 3015 | 0 | 0 | 0 | 0 |
| 3022 | 0 | 0 | 0 | 0 |
| 3023 | 0 | 0 | 0 | 0 |
| 3030 | 0 | 0 | 0 | 0 |
| 3033 | 0 | 0 | 0 | 0 |
| 3037 | 0 | 0 | 0 | 0 |
| 3044 | 0 | 1x 1 | 0 | 0 |
| 3047 | 0 | 0 | 0 | 0 |

Table 20 - Reactions at the subcutaneous injection site
Combat AbaLev and Genesis Injection at standard 1x dose rates in Right neck
* a 1.5 diameter lump within the epidermis due to accidental injection. No reaction to the subcutis injection.

[Table 22]

Table 21 - Reactions at the Subcutaneous injection site - Combat AbaLev at 2x dose rates. As a single injection (2b) or split (2c), left neck

| | Left Neck | Right Neck | Left Neck | Right Neck |
|---|---|---|---|---|
| | | | Day 28 | Day 28 |
| Group 2b. Combat AbaLev Injection. 2x single injection | | | | |
| 3011 | 0 | 0 | 0 | 0 |
| 3015 | 4x2 | 0 | 0 | 0 |
| 3016 | 0 | 0 | 0 | 0 |
| 3019 | 4x2 | 0 | 1x0.5 | 0 |
| 3021 | 0 | 0 | 0 | 0 |
| 3023 | 0 | 0 | 1x 1 | 0 |
| 3039 | 0 | 0 | 0 | 0 |
| 3043 | 20x 7 | 0 | 6x 3 | 0 |
| 3044 | 8x 4 | 0 | 4x3, 1x 1 | 0 |
| 4047 | 0 | 0 | 7x4 | 0 |
| Group 3. Genesis Injection. 2x split as two 1x injections | | | | |
| 3013 | 6x 4 | 0 | 1x1 | 0 |
| 3014 | 0 | 0 | 0 | 0 |
| 3018 | 3.5x 2 | 0* | 0 | 0* |
| 3022 | 2x 1 | 0 | 5x2 | 0 |
| 3024 | 3.5x 3.5 | 0 | 1x1, 2x1 | 0 |
| 3030 | 10x6 | 0 | 2x2,2x2 | 0 |
| 3031 | 0 | 0 | 4x2 | 0 |
| 3033 | 1x 1 | 0 | 0 | 0 |
| 3036 | 7x 4 | 0 | 0 | 0 |
| 3037 | 3x 1 | 0 | 1x1,1,1 | 0 |

Combat AbaLev was well tolerated in calves. Some transient symptoms of salivation and lacrimation were seen at 2 x when given as a single dose, but those receiving 2x as a split dose (two 1x) showed virtually no signs attributable to levamisole with only one animal with some salivation and no transient excitement typical of oral levamisole in calves even at standard dose rates.

Similar results are observed in figure 2 which table is too large to insert in the text of this specification and has to be appended as part of the drawings.

Blood Plasma Profile

The blood (Plasma) profile is shown in Figure 3 and confirms that the levamisole from "Combat AbaLev" is rapidly absorbed, peaking at approximately 4 hours then rapidly eliminated from plasma even within 24 hours. By comparison the abamectin from Combat AbaLev is slowly absorbed and still not has peaked even at 24 hours. Abamectin is expected then to redistribute into fat as it is lipophilic and then slowly release giving it its' persistent effect on incoming parasites. Examples 1 and 2 where the actives were supplied in two different formulations administered at the same time.

**PRIOR ART:**

We have included information from the Ivermectin injection label as it shows the persistent activity against different worm species.

**IVOMEC® (ivermectin) Injection for Cattle, Sheep and Pigs**

A 10g/litre injectable solution of ivermectin for the treatment and control of internal and external parasites of cattle, sheep and pigs. See package insert for complete information.

**Dosage:**

**Cattle:** 1 mL per 50 kg live weight by subcutaneous injection only.
**Sheep:** 0.5 ml per 25 kg live weight by subcutaneous injection only.
**Pigs:** 1 mL per 33 kg live weight by subcutaneous injection only.
IVOMEC Injection given at 1mL/50kg live weight provides effective treatment and control of gastrointestinal roundworms, (including inhibited Ostertagia ostertagi), lungworms, sucking lice and mites of cattle.

**Persistent Activity:**

IVOMEC Injection given at the recommended dosage of 1mL/50kg of live weight effectively controls infections with Ostertagia spp acquired up to at least 14 days after treatment, Cooperia spp. acquired up to at least 7 days after treatment and Dictyocaulus viviparus and Oesophagostomum radiatum acquired up to at least 21 days after treatment.

**Dosage and Administration:**

This pack will treat 10/40/100 x 250kg cattle at the recommended Print as appropriate for pack dose rate of 200 $\mu$g ivermectin per kg live weight. sizes 50, 200 or 500ml.

**SHEEP**

In our trials we have seen and demonstrated this effect with slightly more persistent short acting injectable ivermectin formulations (such as Ivomec injection) and we know that they are acting against Cooperia for only 5-7 days (it has a claim for 7 days).
Virbamec LA with a more persistent injectable ivermectin gave a better response as it acted for around 14 days against Cooperia, and this is like abamectin injection, which is identified as the preferred ML of the invention.
For the Eclipse pour-on we got nearly 21 days against Cooperia but it did not give any better response, in fact less of a production response.
As stated elsewhere we have found that the pour-on formautions do not function as part of the delivery system of this invention. By way of example the Eclipse pour on is not suitable as part of this invention as it delivers levamisole at a higher dose rate.
As we have already discussed, pour-ons while convenient to use, require at least twice the dose of an oral or injection to pass through the skin in effective amounts. There is large between animal variability in how the anthelmintic gets through the skin, and this varies with the animal's individual skin type and also seasonally and also can be influenced by rain. Additionally animals can lick it off and vary the dose that an animal receives. These problems are overcome when delivered either by injection into tissues or orally.
The blood profile of the anthelmintic also varies widely with some ML and BZ pour ons, in some animals blood levels shows the anthelmintic comes in rapidly in high levels over 3-4 days, while in other animals it comes in slowly , and can peak more than 7 days after treatment. This is undesirable for levamisole as it needs rapid high levels to be effective, and pour-ons do not always deliver this and they are influenced by weather, blood supply to the skin and the like.
Also pour-ons deliver the ML in a more erratic fashion and tend to produce quite high tissue residues that deplete slowly and that would be reflected in their persistent activity.

This makes the trial result for the 48 trial animal (above) counter intuitive. It suggests something else other than the anthelmintic is giving these low egg counts and production benefits. We say it is the host/calf doing it when reprogrammed by the method of the invention.

The general rule is that a product with greater persistence would give greater production benefits, and maintain the egg counts lower for longer. What we actually see is the counter intuitive as the weight gains are highest for the Ivermectin injections +levamisole group, not the Eclipse pour on and this was particularly true over the last part of the trial when the persistent activity in the injections had virtually ceased, but Eclipse kept going. We also see the egg counts rising then suddenly dropping in the injectable groups at this time around 42-49 days after treatment exactly as was seen in the first study and this is not seen with the pour on where the egg counts slowly rose over this time.

## ADVANTAGES OF THE PREFERRED EMBODIMENTS

The simultaneous administration of a long acting ivermectin formulation and a much shorter acting levamisole formulation to remove ML resistant Cooperia resulted in the expression of the persistent activity period with complete reductions (100%) in egg count for 4 weeks (28 days), and also the highest weight gains of any combination product tested including Eclipse pour on (abamectin +levamisole).

We have found that by using levamisole base in the formulation rather than levamisole hydrochloride or levamisole phosphate allows a more stable formulation with a higher loading of levamisole. We also found that this levamisole base formulation was less irritant than existing levamisole hydrochloride formulations.

Thus the combination treatment regime of this invention provides a means to minimise drench use by increasing the time between treatment intervals of ruminants. It also stimulates and make use of the hosts own immune system which assists in maintaining the health of the animal and contributes to the increased weight gain compared to the controls. The treatment regime of this invention allows for treatment at between 40 and 60 day intervals compared to recommended treatment intervals of 28 days for Arrest C

This combination treatment has been shown to reduce pasture contamination by breaking the life cycle of the parasite.

Heavy pasture contamination of larvae reduces production, causes higher worm burdens and shortens retreatment intervals. We have shown that the resistant Cooperia larvae from almost all over NZ have sensitive incoming larvae (L3) with the stages that live almost entirely in the animal more resistant (the adult and the L4), it is suspected that this pattern is typical of many other resistant parasites as it is the ones in the animal that are being exposed to the chemical not those on the pasture, and the larval stages change their physiology as they moult.

Even in countries without ML resistant Cooperia the inventive concept applies as even though the ML alone may kill most of the Cooperia, the levamisole and ML will kill more and the time between treatments can be lengthened.

Lungworm larvae are one of the most susceptible of incoming larvae to treatment with Macrocyclic Lactones but single injectable formulation of this invention provides a protection period even longer than the longest ML (moxidectin) protection claim for lungworm. Tests with lungworm larvae did not result in any respiratory disease in Combat AbaLev calves, the theory is that when the larvae did finally get into these Combat AbaLev treated calves (once the long acting ML stopped killing them) it was in small numbers and the animal mounted an immune response to stop more coming in.

In those calves treated with the short acting orals (controls), once the anthelmintic stopped (2-3 days after treatment) the lungworm larvae came in huge numbers and severely damaged the lungs of the calves. Resulting in respiratory distress and deaths occurring approximately just over one month later.

Examples 3 to 6 provide single injectable formulations providing a long acting effect, to enable the animal to better resist incoming larvae and to keep the animal clean of parasites for a longer period of time than with previous injectable formulations, and make it easier for the farmer to administer the treatment. Also by using levamisole base or levamisole phosphate (instead of levamisole hydrochloride) in the examples the formulation is storage stable and is unlikely to result in the levamisole dropping out or crystallising in cold conditions.

By providing the formulation as a non-aqueous formulation, we have found that the formulation is storage stable and can be stored over a relatively wide range of temperatures, although it is recommended that once the container is opened, that the product is stored in a refrigerator, until fully utilised.

Based on our studies we believe that the treatment of this invention results in a delayed host self-treatment effect. Once some worms have re-established the animal appears better able to reduce or stop further incoming worm larvae from establishing, temporarily limiting worm numbers.

At this time the animals acts on the existing resident worms that have reinfected following the anthelmintic treatment with the invention, with a dramatic drop in the faecal worm count, with a positive weight gain/production benefit at the time these events are observed.

It is inferred that given these events the number of worms re-establishing or present in the animal are limited or may even be reduced temporarily, which results in a longer retreatment interval between drenches (40-50 days plus), and also results in a lowering of eggs passed out, therefore resulting in a further benefit of reducing pasture contamination.

It is also noted that a positive response in other conditions such as ringworm is observed at this time, a disease that also requires an optimal immune system to limit and reduce fungal infection of the skin.

The invention thus produces a delayed host self-treatment effect that has beneficial results in reducing the impact of the worms by reducing their egg output, preventing/ reducing worm larval re-establishment, and although we cannot directly show it, we infer results in temporarily reducing worm numbers or reducing the effect of the worm in the host (all of which results in a production/weight gain response, reduced pasture contamination and an animal better able to respond to other conditions including lungworm and also ringworm).

We have also described this effect "as allowing the animal's immune system to recognise incoming larvae as foreign" but it is recognised that the way in which the animal may respond and achieve this may be complex, and may include the host releasing non-specific chemicals which act on the established worm or act to inhibit, kill or prevent reestablishment of the incoming larvae.

## VARIATIONS

We have provided examples of only a few of the possible macrocyclic lactones. Any of the existing, or future macrocyclic lactones could be used in combination with levamisole in the formulations of this invention, and it is also possible that other actives could be used in such an injectable formulation.

Throughout the description of this specification, the word "comprise" and variations of that word such as "comprising" and "comprises", are not intended to exclude other additives, components, integers or steps.

It will of course be realised that while the foregoing has been given by way of illustrative example of this invention, all such and other modifications and variations thereto as would be apparent to persons skilled in the art are deemed to fall within the broad scope and ambit of this invention as is hereinbefore described.

## Industrial Applicability

The invention provides a novel treatment strategy for ruminants especially cattle and sheep, and has been shown to provide an increased weight gain especially in herds of cattle. It also provides novel injectable formulations based on this treatment strategy, and allows for a longer period between treatments than existing anthelmintics. For example this invention allows for treatment of the animal at between 40 and 60 day intervals compared to recommended treatment intervals of 28 days for Arrest C.

## Claims

1. An anthelmintic formulation combining

- a short acting injectable anthelmintic formulation B comprising an initial dose of levamisole base, levamisole phosphate or levamisole hydrochloride at 3 to 9 mg levamisole base equivalent per kg of the animal's live weight, said formulation B substantially eliminates the initial parasite population of a ruminant animal and
- a long acting injectable anthelmintic formulation A comprising an initial dose of 0,1 to 0,5 mg of one or more macrocyclic lactones (MLs) per kg of the animal's live weight, said formulation A being capable of removing or killing incoming larvae for a sufficiently long period to allow the host to recognise incoming larvae as foreign and mount an immune response to the incoming larvae,

For use in the treatment of internal parasites infestations of a young ruminant animal at intervals of 40 to 60 days by allowing the animal's immune system to recognise incoming larvae as foreign.

2. The anthelmintic formulation as claimed in claim 1 for use as claimed in claim 1, wherein the two formulations A and B are separate injectable formulations administered to the animal, and wherein formulation B is injected into the animal at the same time as or before formulation A.

3. The anthelmintic formulation as claimed in claim 1 or in claim 2 for use as claimed in claim 1, wherein formulations A and B are delivered simultaneously to the animal in a combined injectable formulation.

4. The anthelmintic formulation as claimed in anyone of claims 1 to 3 for use as claimed in claim 1, wherein the short acting anthelmintic formulation B is capable of peaking within 1-8 hours, and being substantially eliminated from the ruminant within 48 hours of treatment.

**5.** The anthelmintic formulation as claimed in anyone of claims 1 to 4 for use as claimed in claim 1, wherein the long acting anthelmintic formulation A and is capable of providing a persistent activity of anthelmintic A from 10-14 days.

**6.** The anthelmintic formulation as claimed in anyone of claims 1 to 5 for use as claimed in claim 1, wherein the animal is treated with about 7.5mg/kg to 8mg/kg of levamisole base on a live weight basis.

**Patentansprüche**

**1.** Anthelmintische Formulierung, kombinierend:

- eine kurz wirkende injizierbare anthelmintische Formulierung B, umfassend eine Ausgangsdosis von Levamisol-Base, Levamisol-Phosphat oder Levamisol-Hydrochlorid bei 3 bis 9 mg Levamisol-Base-Äquivalent pro kg Lebendgewicht des Tieres, wobei die Formulierung B die anfängliche Parasitenpopulation eines Wiederkäuers im Wesentlichen eliminiert, und
- eine lang wirkende injizierbare anthelmintische Formulierung A, umfassend eine Anfangsdosis von 0,1 bis 0,5 mg eines oder mehrerer makrocyclischer Lactone (MLs) pro kg Lebendgewicht des Tieres, wobei die Formulierung A in der Lage ist, eindringende Larven für einen Zeitraum zu entfernen oder zu töten, der lange genug ist, um dem Wirt zu ermöglichen, eindringende Larven als fremd zu erkennen und eine Immunantwort gegen die eindringenden Larven aufzubauen,

zur Verwendung bei der Behandlung von Endoparasitenbefall eines jungen Wiederkäuers in Intervallen von 40 bis 60 Tagen, indem dem Immunsystem des Tieres ermöglicht wird, eindringende Larven als fremd zu erkennen.

**2.** Anthelmintische Formulierung nach Anspruch 1 zur Verwendung nach Anspruch 1, wobei die beiden Formulierungen A und B getrennte injizierbare Formulierungen sind, die dem Tier verabreicht werden, und wobei die Formulierung B dem Tier gleichzeitig mit oder vor der Formulierung A injiziert wird.

**3.** Anthelmintische Formulierung nach Anspruch 1 oder Anspruch 2 zur Verwendung nach Anspruch 1, wobei die Formulierungen A und B dem Tier in einer kombinierten injizierbaren Formulierung gleichzeitig verabreicht werden.

**4.** Anthelmintische Formulierung nach einem der Ansprüche 1 bis 3 zur Verwendung nach Anspruch 1, wobei die kurz wirkende anthelmintische Formulierung B innerhalb von 1 bis 8 Stunden ihre stärkste Wirkung erzielen und innerhalb von 48 Stunden ab der Behandlung vom Wiederkäuer im Wesentlichen ausgeschieden werden kann.

**5.** Anthelmintische Formulierung nach einem der Ansprüche 1 bis 4 zur Verwendung nach Anspruch 1, wobei die lang wirkende anthelmintische Formulierung A nach 10 bis 14 Tagen eine persistente Aktivität des Anthelminthikums A bereitstellen kann.

**6.** Anthelmintische Formulierung nach einem der Ansprüche 1 bis 5 zur Verwendung nach Anspruch 1, wobei das Tier mit etwa 7,5 mg/kg bis 8 mg/kg Levamisol-Base bezogen auf das Lebendgewicht behandelt wird.

**Revendications**

**1.** Formulation antihelminthique combinant

- une formulation antihelminthique injectable à action brève B comprenant une dose initiale de lévamisole base, de phosphate de lévamisole ou de chlorhydrate de lévamisole dans les 3 à 9 mg d'équivalent lévamisole base par kg du poids vif de l'animal, ladite formulation B élimine sensiblement la population parasitaire initiale d'un animal ruminant et
- une formulation antihelminthique injectable à action prolongée A comprenant une dose initiale de 0,1 à 0,5 mg d'une ou de plusieurs lactones macrocycliques (ML) par kg du poids vif de l'animal, ladite formulation A étant apte à supprimer ou à tuer les nouvelles larves pendant une période suffisamment longue pour permettre à l'hôte de reconnaître les nouvelles larves comme corps étrangers et de développer une réponse immunitaire contre les nouvelles larves,

Destinée à être utilisée dans le traitement d'infestations parasitaires internes d'un jeune animal ruminant selon des intervalles de 40 à 60 jours en permettant au système immunitaire de l'animal de reconnaître les nouvelles larves comme corps étrangers.

2. Formulation antihelminthique selon la revendication 1 destinée à une utilisation telle que décrite en revendication 1, dans laquelle les deux formulations A et B sont des formulations injectables distinctes administrées à l'animal, et dans laquelle la formulation B est injectée dans l'animal au même moment que ou avant la formulation A.

3. Formulation antihelminthique selon la revendication 1 ou la revendication 2 destinée à une utilisation telle que décrite en revendication 1, dans laquelle les formulations A et B sont administrées simultanément à l'animal dans une formulation injectable combinée.

4. Formulation antihelminthique selon l'une quelconque des revendications 1 à 3 destinée à une utilisation telle que décrite en revendication 1, dans laquelle la formulation antihelminthique injectable à action brève B est apte à atteindre son maximum dans les 1 à 8 heures, et étant sensiblement éliminée du ruminant dans les 48 heures de traitement.

5. Formulation antihelminthique selon l'une quelconque des revendications 1 à 4 destinée à une utilisation telle que décrite en revendication 1, dans laquelle la formulation antihelminthique injectable à action prolongée A est apte à fournir une activité persistante de l'antihelminthique A de 10 à 14 jours.

6. Formulation antihelminthique selon l'une quelconque des revendications 1 à 5 destinée à une utilisation telle que décrite en revendication 1, dans laquelle l'animal est traité avec environ 7,5 mg/kg à 8 mg/kg de lévamisole base sur la base d'un poids vif.

Figure 1 – Arithmetic mean faecal egg counts

Figure 2 - Total larvae recovered in culture per 25g by treatment group

# FIGURE 3

Mean Levamisole and Abamectin B1a Concentration in Plasma

# FIGURE 4

| Group | Tag No- Left | Sex | Breed | Weight 1 kg | Weight 2 kg | Weight 3 kg | Weigh Diff 3-2 kg | Condition score | FEC (EPG) | Skin | Comment | Group |
|---|---|---|---|---|---|---|---|---|---|---|---|---|
| | | | | 15-Jun | 26-Jun | 18-Jul | (22 days) | | 18-Jul | | | |
| Controls | | | | | | | | | | | | |
| 1c | 58 | F | Fr/H. Red&W | 74.5 | 78.5 | 83.5 | 5.0 | 2.0 | 450 | 0 | | 1c |
| 1c | 3102 | M | Fr.J.Black | 78 | 88.0 | 98.5 | 10.5 | 2.0 | 300 | 0 | | 1c |
| 1c | 3105 | M | Fr.J.Black | 72 | 80.5 | 88.5 | 8.0 | 3.0 | 1000 | 2 | ++ Crusts around base | 1c |
| 1c | 3112 | M | Fr.J.Black | 67 | 72.0 | 79.5 | 7.5 | 4.0 | 500 | 2 | +++ Crusts base of tail | 1c |
| 1c | 3113 | M | Fr/H. Red&W | 82.5 | 90.5 | 101.5 | 11.0 | 2.0 | 400 | 0 | | 1c |
| | MEAN | | | 74.8 | 81.9 | 90.3 | 8.4 | | 530 | | | |
| | | | | | | | | | | | | |
| 3a | 3106 | M | Fr.J.Black | 93 | 101.5 | 119.0 | 17.5 | 2.0 | 0 | 0 | 0 | 3a |
| 3a | 3114 | M | Fr. Block&W | 69 | 77.5 | 91.5 | 14.0 | 3.0 | 0 | 1 | + scurf at base of tail | 3a |
| 3a | 3116 | M | Fr.J.Black | 72.5 | 79.0 | 94.0 | 15.0 | 2.0 | 0 | 0 | | 3a |
| 3a | 3119 | M | Fr.J.Black | 77 | 84.5 | 98 | 13.5 | 2.0 | 0 | 1 | + scurf at base of tail | 3a |
| 3a | 3124 | M | Fd.J. Black | 60 | 67.5 | 79.5 | 12.0 | 2.0 | 0 | 1 | + scurf at base of tail | 3a |
| | MEAN | | | 74.3 | 82.0 | 96.4 | 14.4 | | 0 | | | |
| | | | | | | | | | | | | |
| 3b | 3103 | M | Fr.J.Black | 87.0 | 99.5 | 117.5 | 18.0 | 2.0 | 0 | 1 | + scurf at base of tail | 3b |
| 3b | 3104 | M | Fr.J.Black | 70 | 76.5 | 90.5 | 14.0 | 2.0 | 0 | 0 | 0 | 3b |
| 3b | 3107 | M | Fr.J.Black | 66 | 75.0 | 84.0 | 9.0 | 2.0 | 0 | 1 | + scurf at base of tail | 3b |
| 3b | 3117 | M | Fr.J.Black | 76 | 84.0 | 98.0 | 14.0 | 2.0 | 0 | 1 | + scurf at base of tail | 3b |
| 3b | 3118 | M | Fd.J.Black | 73.5 | 81.0 | 99.0 | 18.0 | 2.0 | 0 | 0 | 0 | 3b |
| | MEAN | | | 74.5 | 83.2 | 97.8 | 14.6 | | 0 | | | |

Extra control

| 3111 | M | Fr/H. Red&W | 59 | 65.5 | 72 | 6.5 | 4 | 600 | 0 | 0 |
|---|---|---|---|---|---|---|---|---|---|---|

| Body Score | |
|---|---|
| 1= Excellent | |
| 2=Good | |
| 3= Moderate-light | |
| 4 = Poor | |
| 5 = Very poor | |

| Skin Grade | |
|---|---|
| 0 = no lesions | |
| 1 = scurf | |
| 2 = crusts/scabs | |
| mild + | |
| moderate ++ | |
| marked +++ | |
| severe ++++ | |

Treatment

| Controls | Treated with Arrest C (Albendazole + Levamisole oral on 21 May, approximately 59 days previously at end of study at 18 July | | | |
|---|---|---|---|---|
| 3108 and 3103 | Treated with 3x Combat AbaLev on 16 June or 32 days previously at the end of study at 18 July | | | |
| All other calves | Treated with 3x Combat AbaLev on 27 June or 21 days previously at the end of study at 18 July | | | |
| | | | | |

Adult nematodes in the digestive tract of sheep lay their eggs.

Eggs passed onto pasture in manure.

Infective larvae are ingested by grazing sheep

Eggs hatch, and larvae develop to infective 3rd stage in soil and manure.

# FIGURE 5

**EP 2 744 495 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4159337 A
- US 4166858 A
- WO 19941028887 A
- WO 2004069242 A
- NZ 336139
- NZ 336213
- NZ 336814
- WO 2004009080 A
- WO 0074489 A